# EUROPEAN PATENT APPLICATION

(11) **EP 4 285 899 A1**
(43) Date of publication of application: **06.12.2023**
(21) Application number: 22745081.4
(22) Date of filing: 17.01.2022
(51) Int. Cl.: A61K 31/437, C07D 471/04, C07D 487/00, A61P 37/06, A61P 37/08, A61P 19/02

(54) **ORAL PREPARATION CONTAINING JAK INHIBITOR OR SALT THEREOF OR CRYSTAL FORM THEREOF, PREPARATION METHOD THEREFOR, AND APPLICATION THEREOF**

(30) Priority: 29.01.2021 CN 202110114769
(71) Applicant: Zhuhai United Laboratories Co., Ltd., Zhuhai, Guangdong 519040 (CN)
(72) Inventor: WANG, Zheng, Zhongshan, Guangdong 528467 (CN); GAO, Peng, Zhongshan, Guangdong 528467 (CN); YU, Tingting, Zhongshan, Guangdong 528467 (CN); HE, Yujun, Zhongshan, Guangdong 528467 (CN); SHEN, Meiyue, Zhongshan, Guangdong 528467 (CN); MU, Liwei, Zhongshan, Guangdong 528467 (CN); LIU, Jingjing, Zhongshan, Guangdong 528467 (CN); SHI, Lixiu, Zhongshan, Guangdong 528467 (CN); WANG, Degang, Zhongshan, Guangdong 528467 (CN)
(74) Representative: Kröncke, Rolf
(86) International application number: PCT/CN2022/072415
(87) International publication number: WO 2022/161205

(57) **Abstract**

The present application provides an oral preparation, specifically including a JAK inhibitor and pharmaceutical excipients, wherein the JAK inhibitor includes a compound of formula (I), an isomer thereof, pharmaceutically acceptable salts thereof, or their crystal form. The oral preparation of the present application has the characteristic of rapid dissolution. The oral preparation process of the present application is simple and suitable for large-scale industrial production.

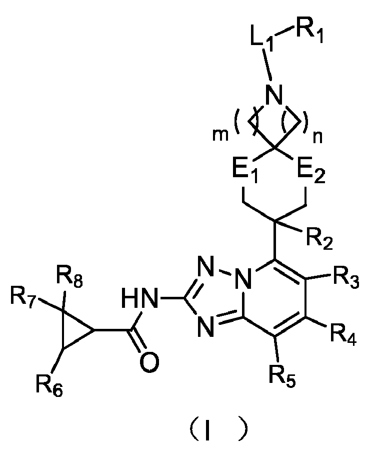

## Description

This application claims a priority to CN202110114769.6, filed on January 29, 2021.

### TECHNICAL FIELD

The present application belongs to the field of pharmaceutical preparations, and, in particular,relates to an oral preparation of a class of [1,2,4] triazolo [1,5-a] pyridine compounds and isomers or pharmaceutically acceptable salts or crystal forms thereof, as well as preparation method and application thereof. The oral preparation has the characteristics of rapid dissolution.

### BACKGROUND ART

JAK kinases are a family of intracellular non-receptor tyrosine kinases. The kinase family has four members: JAK1, JAK2, JAK3, and TYK2. JAK1, JAK2, and TYK2 are all expressed in human histiocyte, and JAK3 is mainly expressed in hematopoietic histiocyte. JAK belongs to a tyrosine kinase family involved in inflammation, autoimmune diseases, proliferative diseases, transplant rejection (or graft versus host disease), diseases involving impaired cartilage turnover, congenital cartilage malformations, and/or diseases related to excessive IL6 secretion. Research has confirmed that inhibiting the JAK signaling pathway is believed to regulate multiple signaling pathways related to inflammation, autoimmune diseases, proliferative diseases, transplant rejection, diseases involving impaired cartilage turnover, congenital cartilage malformations, and/or diseases related to excessive IL-6 secretion.

The four family members of JAK kinases selectively bind to different cytokine receptors and play different physiological roles. The downstream of JAKs is the Signal Transducers and Activators of Transcription (STAT) family. The JAK-STAT pathway can transmit extracellular signals from various cytokines, interferons, most interleukins, and endocrine factors to the cell nucleus, and is responsible for the expression of protein coding genes. When cytokines bind to their receptors, members of the JAK family undergo self phosphorylation and/or mutual phosphorylation, followed by phosphorylation of STATs and migration to the nucleus to regulate transcription.

The JAK-STAT pathway is one of the main research directions in the pathogenesis of rheumatoid arthritis (RA), and studies have shown that IFN- γ, TNF- α, IL-1 β, IL-2, IL-4, IL-6, IL-7, IL-9, IL-10, IL-15, IL-17, IL-21, and many other cytokines play an important role in the pathogenesis of RA by influencing the JAK-STAT pathway. This signaling pathway is continuously activated during RA, participating in the proliferation of synovial cells and the release of inflammatory cytokines. Therefore, targeted blockade of the JAK-STAT pathway can achieve the goal of regulating cell activity and improving the pathological process of RA.

WO/2020/038457 discloses a series of JAK kinase inhibitors that satisfy the general formula I of [1,2,4]triazolo[1,5-a] pyridine compounds and isomers or pharmaceutically acceptable salts thereof:

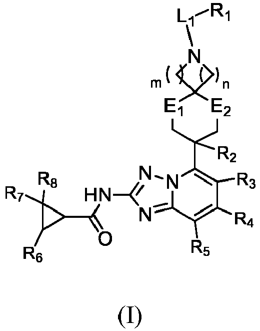

It is known that it is a small molecule JAK kinase inhibitor with significant JAK kinase inhibitory activity and high selectivity, and it is expected that these compounds may be used for the treatment of rheumatoid arthritis. The oral preparations of the JAK kinase inhibitor has not yet been publicly disclosed.

These compounds include compounds as shown in formula II, with the chemical name (S)-N-(5-(2-(2,2-difluorocyclopropane carbonyl)-2-azaspiro[3.5]non-7-yl)-[1,2,4]triazole[1,5-a] pyridin-2-yl) cyclopropane formamide:

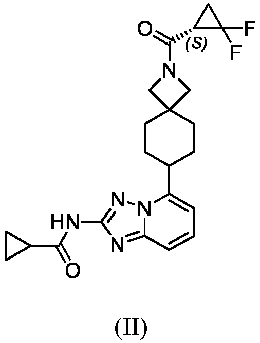

The above literature further does not disclose how to obtain rapidly and completely dissolved oral preparations. Therefore, further research is needed to discover well dissolved and stable oral preparations, which have a simple preparation process and are suitable for industrial production.

### SUMMARY

A purpose of the present application is to provide an oral preparation with rapid dissolution and good stability, and the preparation process of the oral preparation is simple, suitable for industrial large-scale production.

The present application provides an oral preparation comprising a JAK inhibitor and a pharmaceutical excipient, wherein the JAK inhibitor includes a compound of formula (I), an isomer thereof, pharmaceutically acceptable salts thereof, or crystal form thereof: 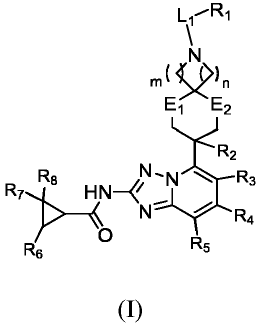 wherein,
E₁ and E₂ are independently selected from single bond, -CH₂- or -(CH₂)₂-, respectively;
L₁ is selected from a group consisting of single bond, - (CH₂)_{g}-, -C(=O)- or -C(=O)-(CH₂)ₕ-;
m is 1 or 2;
n is 1 or 2;
g is 1, 2 or 3;
h is 1, 2 or 3;
R₁ is selected from H, CN, C₁₋₆ alkyl or 3-6-membered cycloalkyl, wherein the C₁₋₆ alkyl and 3-6-membered cycloalkyl are optionally substituted by 1, 2 or 3 Rₐ;
R₂ is selected from H, F, Cl, Br, I or C₁₋₃ alkyl, wherein the C₁₋₃ alkyl is optionally substituted by 1, 2 or 3 R_{b};
R₃, R₄ and R₅ are independently selected from H, F, Cl, Br, I or C₁₋₃ alkyl, respectively, wherein the C₁₋₃ alkyl is optionally substituted by 1, 2 or 3 R_{c};
R₆, R₇ and R₈ are independently selected from H, F, Cl, Br, I or C₁₋₃ alkyl, respectively, wherein the C₁₋₃ alkyl is optionally substituted by 1, 2 or 3 R_{d};
Each Rₐ is independently selected from H, F, Cl, Br, I, CN or C₁₋₃ alkyl, respectively, wherein the C₁₋₃ alkyl is optionally substituted by 1, 2 or 3 R;
Each R_{b} is independently selected from F, Cl, Br or I;
Each R_{c} is independently selected from F, Cl, Br or I;
Each R_{d} is independently selected from F, Cl, Br or I; and
Each R is independently selected from F, Cl, Br or I.

The pharmaceutical excipients include a filler, a disintegrant, an adhesive, a lubricant or a surfactant, or a combination of two or more thereof.

In the present application, as one of the embodiments, the compound of formula (I), isomers thereof, or pharmaceutically acceptable salts thereof, wherein each Rₐ is independently selected from H, F, Cl, Br, I, or CN.

In the present application, as one of the embodiments, the compound of formula (I), isomers thereof, or pharmaceutically acceptable salts thereof, wherein R₁ is selected from H, CN, C₁₋₃ alkyl or 3-5-membered cycloalkyl, wherein the C₁₋₃ alkyl and 3-5-membered cycloalkyl are optionally substituted by 1, 2, or 3 Ra.

In the present application, as one of the embodiments, the compound of formula (I), isomers thereof, or pharmaceutically acceptable salts thereof, wherein R₁ is selected from H, CN, CH3, 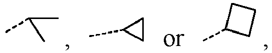 wherein CH₃, 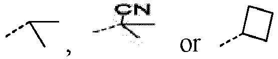 is optionally substituted by 1, 2, or 3 Ra.

In the present application, as one of the embodiments, the compound of formula (I), isomers thereof, or pharmaceutically acceptable salts thereof, wherein R₁ is selected from a H, CN, CF₃, CHF₂,

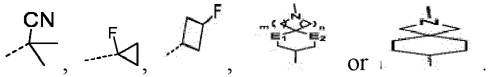

In the present application, as one of the embodiments, the compound of formula (I), isomers thereof, or pharmaceutically acceptable salts thereof, wherein R2 is selected from H, F, Cl, Br, or I.

In the present application, as one of the embodiments, the compound of formula (I), isomers thereof, or pharmaceutically acceptable salts thereof, wherein R₃, R₄, and R₅ are independently selected from H, F, Cl, Br, or I.

In the present application, as one of the embodiments, the compound of formula (I), isomers thereof, or pharmaceutically acceptable salts thereof, wherein R₆, R₇, and R₈ are independently selected H, F, Cl, Br, or I.

In the present application, as one of the embodiments, the compound of formula (I), isomers thereof, or pharmaceutically acceptable salts thereof, wherein L₁ is selected from a single bond, -CH₂-, -(CH₂)₂-, -C(=O)- or -C(=O)-(CH₂)-.

In the present application, as one of the embodiments, the compound of formula (I), isomers thereof, or pharmaceutically acceptable salts thereof, wherein the structural unit 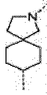 is selected from

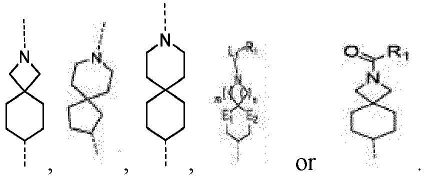

In the present application, as one of the embodiments, the compound of formula (I), isomers thereof, or pharmaceutically acceptable salts thereof, wherein the structural unit 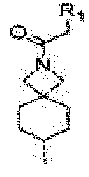 is selected from

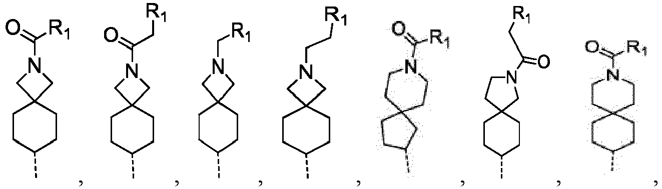

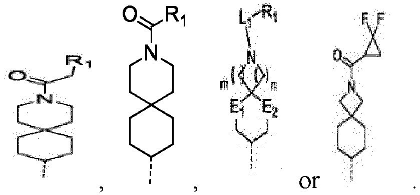

In the present application, as one of the embodiments, the compound of formula (I), isomers thereof, or pharmaceutically acceptable salts thereof, wherein the structural unit 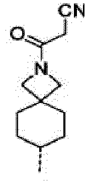 is selected from 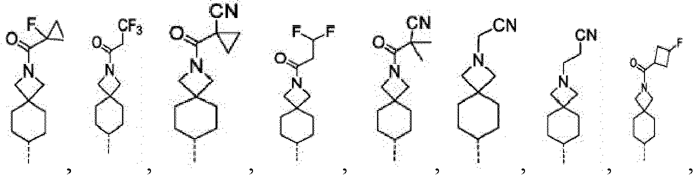 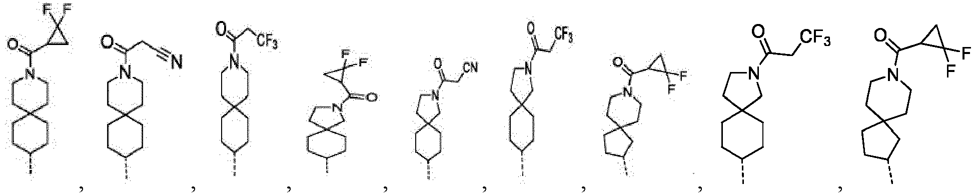 or

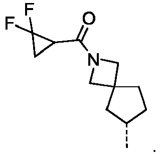

In the present application, as one of the embodiments, the compound of formula (I), isomers thereof, or pharmaceutically acceptable salts thereof, is selected 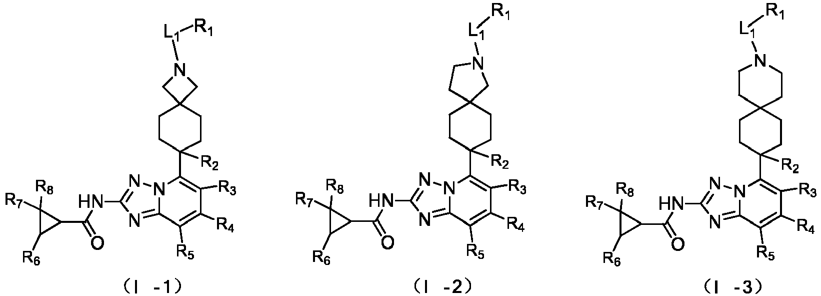 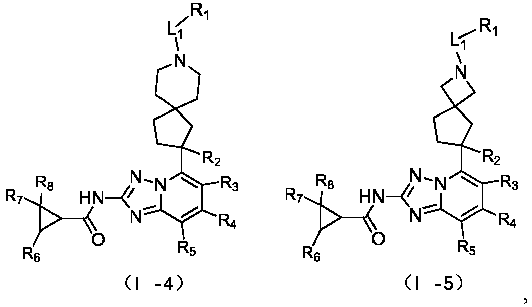 Wherein
L₁ is defined in claim 1 or 9;
R₁ is defined in claims 1-5;
R₂ is defined in claim 1 or 6;
R₃, R₄, and R₅ is defined in claim 1 or 7;
R₆, R₇, and R₈ is defined in claim 1 or 8.

In the present application, as one of the embodiments, the compound of formula (I), isomers thereof, or pharmaceutically acceptable salts thereof, is selected from 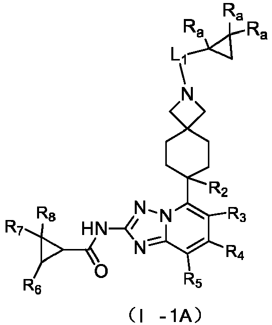 wherein,
L₁ is defined in claim 1 or 9;
R₁ is defined in claim 1 or 2;
R₂ is defined in claim 1 or 6;
R₃, R₄, and R₅ are defined in claim 1 or 7;
R₆, R₇, and R₈ are defined in claim 1 or 8.

In the present application, as one of the embodiments, JAK inhibitors include the following compounds, their isomers, or pharmaceutically acceptable salts thereof

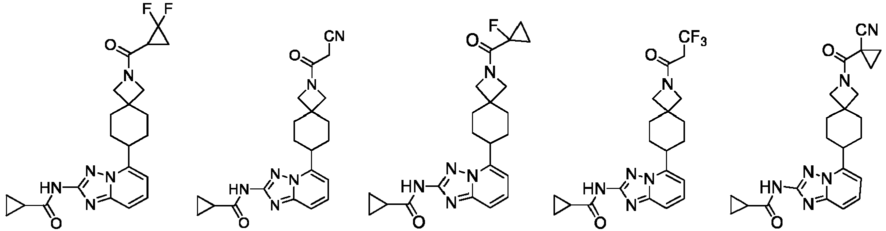

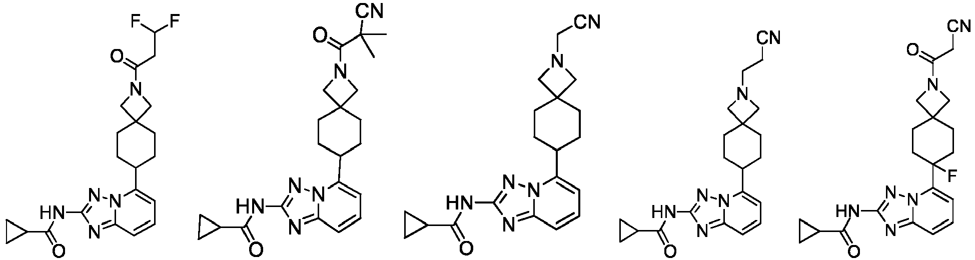

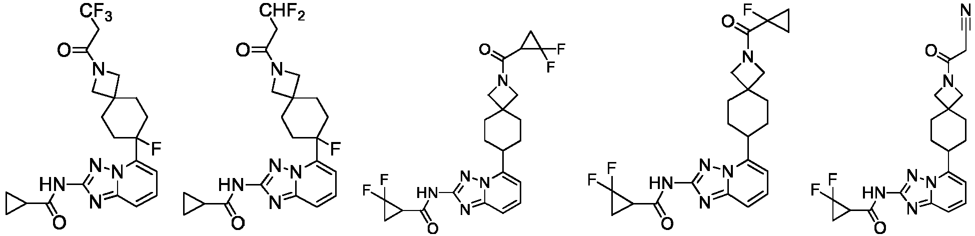

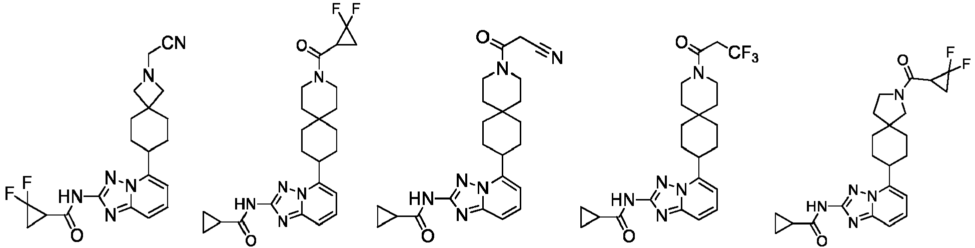

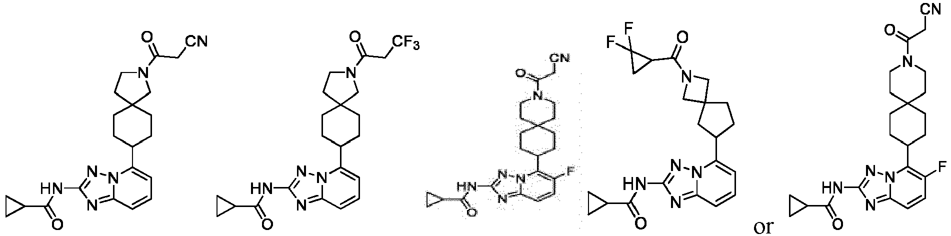

In the present application, as one of the embodiments, the compound of formula (I), isomers thereof, or pharmaceutically acceptable salts thereof, is selected

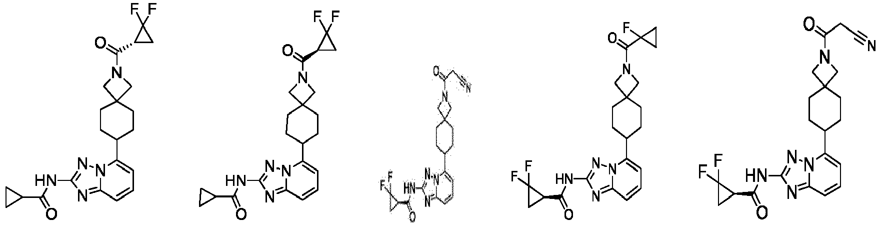

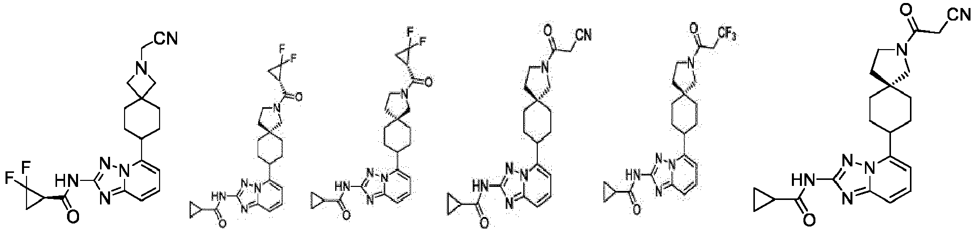

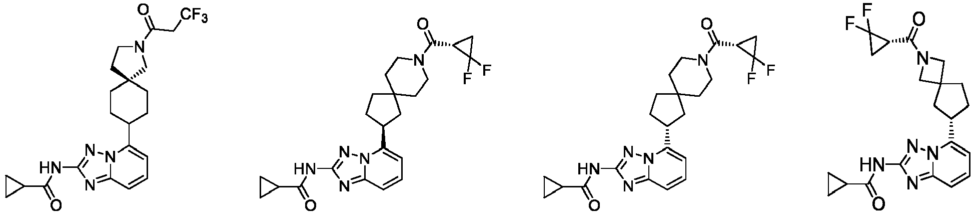

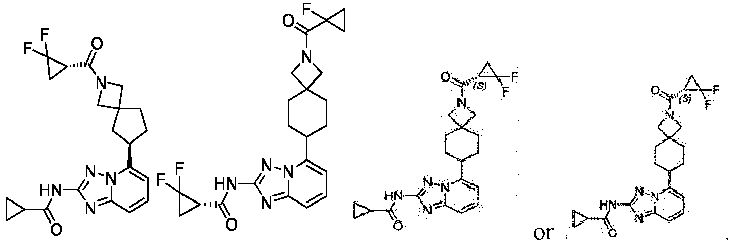

In the present application, as one of the embodiments, the oral preparation includes one or more fillers.

In the present application, as one of the embodiments, the oral preparation includes one or more disintegrants.

In the present application, as one of the embodiments, the oral preparation includes one or more adhesives.

In the present application, as one of the embodiments, the oral preparation further includes one or more lubricants.

In the present application, as one of the embodiments, the oral preparation further includes one or more surfactants.

In the present application, as one of the embodiments, the oral preparation includes one or more fillers and one or more disintegrants.

In the present application, as one of the embodiments, the oral preparation includes one or more fillers, one or more disintegrants, and one or more adhesives.

In the present application, as one of the embodiments, the oral preparation includes one or more fillers, one or more disintegrants, one or more adhesives, and one or more lubricants.

In the present application, as one of the embodiments, the oral preparation includes one or more fillers, one or more disintegrants, one or more adhesives, one or more lubricants, and one or more surfactants.

In the present application, as one of the embodiments, the filler is selected from microcrystalline cellulose, lactose, pre-gelatinized starch or anhydrous dicalcium phosphate, or a combination of two or more of them.

In the present application, as one of the embodiments, the disintegrant is selected from cross-linked carboxymethyl cellulose sodium, carboxymethyl starch sodium, cross-linked polyvinylketone or dry starch, or a combination of two or more of them.

In the present application, as one of the embodiments, the adhesive is selected from a carboxymethyl cellulose, hydroxypropyl cellulose, polyvinylketone, methyl cellulose, or ethyl cellulose, or a combination of two or more of them.

In the present application, as one of the embodiments, the lubricant is selected from magnesium stearate, micronized silica gel, talc powder, hydrogenated vegetable oil, polyethylene glycol or sodium dodecyl sulfate, or a combination of two or more of them.

In the present application, as one of the embodiments, the surfactant is sodium dodecyl sulfate.

The present application provides an oral preparation comprising compound (S)-N-(5-(2-(2,2-difluorocyclopropane carbonyl)-2-azaspiro[3.5]non 7-yl)-[1,2,4]triazole[1,5-a]pyridin-2-yl) cyclopropane formamide of formula (II) or pharmaceutically acceptable salts thereof, as well as one or more fillers, one or more disintegrants, one or more adhesives, one or more lubricants, and one or more surfactants.

In the present application, as one of the embodiments, crystal form A of the compound of formula (II) or pharmaceutically acceptable salts thereof 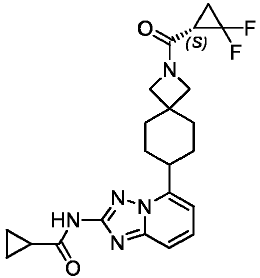 is A, B, C, or crystal form D.

In the present application, as one of the embodiments, the analytical data of the A-crystal form XRPD diagram of the compound of formula (II) is as follows:

| **No.** | **2θ angle (°)** | **Relative strength (%)** | **No.** | **2θ angle (°)** | **Relative strength (%)** |
|---|---|---|---|---|---|
| 1 | 6.91 | 100.0 | 18 | 25.88 | 8.3 |
| 2 | 10.34 | 13.7 | 19 | 26.18 | 4.9 |
| 3 | 12.21 | 64.7 | 20 | 26.93 | 2.8 |
| 4 | 13.69 | 23.1 | 21 | 27.52 | 18.9 |
| 5 | 14.44 | 9.8 | 22 | 28.73 | 2.6 |
| 6 | 16.11 | 3.1 | 23 | 29.12 | 8.2 |
| 7 | 17.44 | 12.2 | 24 | 29.92 | 6.8 |
| 8 | 18.11 | 18.7 | 25 | 31.56 | 6.9 |
| 9 | 18.76 | 11.7 | 26 | 31.86 | 9.6 |
| 10 | 19.06 | 76.7 | 27 | 32.43 | 6.0 |
| 11 | 19.86 | 32.6 | 28 | 32.64 | 7.2 |
| 12 | 20.59 | 28.8 | 29 | 33.04 | 2.5 |
| 13 | 22.06 | 24.8 | 30 | 33.30 | 2.3 |
| 14 | 22.63 | 6.8 | 31 | 34.52 | 10.0 |
| 15 | 23.49 | 2.4 | 32 | 35.21 | 3.9 |
| 16 | 24.46 | 12.0 | 33 | 35.56 | 2.8 |
| 17 | 25.27 | 4.6 | | | |

In the present application, as one of the embodiments, the analytical data of the B-crystal form XRPD diagram of the compound of formula (II) is as follows:

| **No.** | **2θ angle (°)** | **Relative strength (%)** | **No.** | **2θ angle (°)** | **Relative strength (%)** |
|---|---|---|---|---|---|
| 1 | 5.13 | 65.7 | 20 | 26.06 | 9.0 |
| 2 | 7.34 | 43.4 | 21 | 26.61 | 4.1 |
| 3 | 10.14 | 27.3 | 22 | 27.03 | 7.2 |
| 4 | 10.56 | 23.7 | 23 | 27.69 | 2.0 |
| 5 | 11.46 | 13.7 | 24 | 28.45 | 2.5 |
| 6 | 11.72 | 42.7 | 25 | 28.97 | 5.5 |
| 7 | 14.64 | 2.1 | 26 | 29.49 | 8.8 |
| 8 | 16.67 | 27.0 | 27 | 29.81 | 2.8 |
| 9 | 16.86 | 13.6 | 28 | 30.41 | 6.7 |
| 10 | 18.84 | 22.5 | 29 | 30.72 | 3.4 |
| 11 | 19.14 | 55.0 | 30 | 31.15 | 3.2 |
| 12 | 20.31 | 3.7 | 31 | 32.00 | 3.1 |
| 13 | 21.18 | 100.0 | 32 | 32.35 | 3.0 |
| 14 | 21.78 | 20.5 | 33 | 33.34 | 3.6 |
| 15 | 22.03 | 9.3 | 34 | 33.65 | 2.3 |
| 16 | 22.54 | 14.2 | 35 | 37.00 | 2.5 |
| 17 | 22.96 | 13.2 | 36 | 38.60 | 2.8 |
| 18 | 24.52 | 4.1 | 37 | 39.66 | 2.1 |
| 19 | 25.27 | 11.5 | | | |

In the present application, as one of the embodiments, the analytical data of the C-crystal form XRPD diagram of the compound of formula (II) is as follows:

| **No.** | **2θ angle (°)** | **Relative strength (%)** | **No.** | **2θ angle (°)** | **Relative strength (%)** |
|---|---|---|---|---|---|
| 1 | 5.76 | 19.3 | 8 | 18.66 | 29.0 |
| 2 | 8.92 | 31.2 | 9 | 19.17 | 22.1 |
| 3 | 11.50 | 13.9 | 10 | 20.26 | 100.0 |
| 4 | 14.28 | 11.4 | 11 | 22.98 | 6.2 |
| 5 | 16.35 | 23.6 | 12 | 24.79 | 24.0 |
| 6 | 17.54 | 11.4 | 13 | 29.77 | 5.1 |
| 7 | 17.99 | 7.5 | | | |

In the present application, as one of the embodiments, the analytical data of the D-crystal form XRPD diagram of the compound of formula (II) is as follows:

| **No.** | **2θ angle (°)** | **Relative strength (%)** | **No.** | **2θ angle (°)** | **Relative strength (%)** |
|---|---|---|---|---|---|
| 1 | 7.12 | 100.0 | 14 | 21.42 | 44.1 |
| 2 | 10.28 | 12.3 | 15 | 21.84 | 7.9 |
| 3 | 10.68 | 2.7 | 16 | 23.95 | 3.0 |
| 4 | 12.45 | 36.0 | 17 | 24.50 | 5.1 |
| 5 | 13.04 | 7.2 | 18 | 25.74 | 4.3 |
| 6 | 14.28 | 26.7 | 19 | 26.69 | 4.9 |
| 7 | 14.64 | 30.4 | 20 | 27.24 | 2.5 |
| 8 | 16.06 | 3.1 | 21 | 28.72 | 17.0 |
| 9 | 17.30 | 7.2 | 22 | 29.49 | 5.0 |
| 10 | 17.50 | 10.8 | 23 | 31.80 | 4.3 |
| 11 | 18.31 | 16.1 | 24 | 34.05 | 7.8 |
| 12 | 20.54 | 77.4 | 25 | 36.04 | 6.1 |
| 13 | 20.93 | 20.5 | | | |

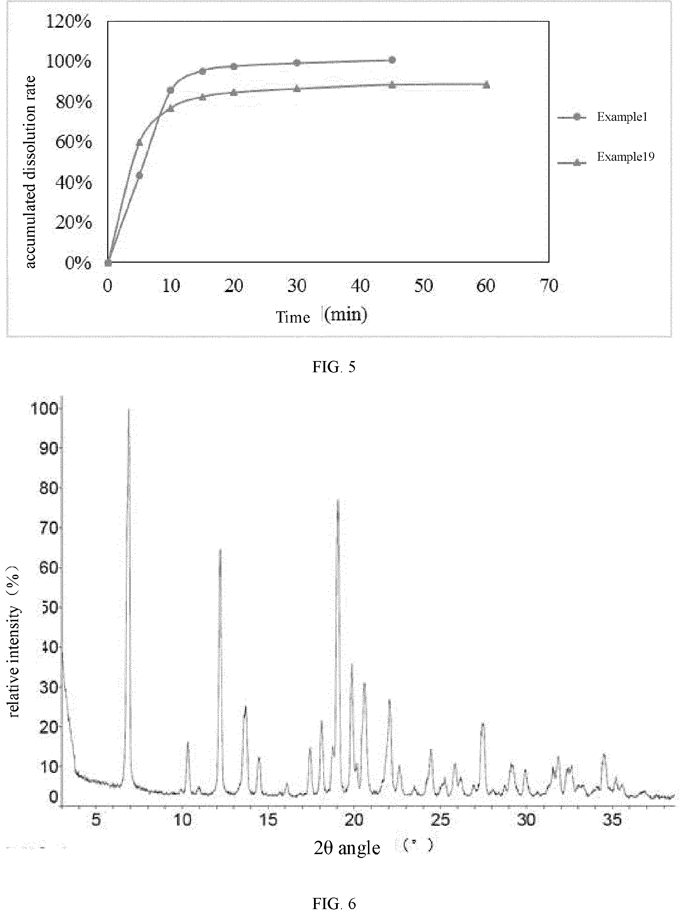

In the present application, as one of the embodiments, the XRPD diagram of the crystal form A of the compound of formula (II) is shown in FIG. 6.

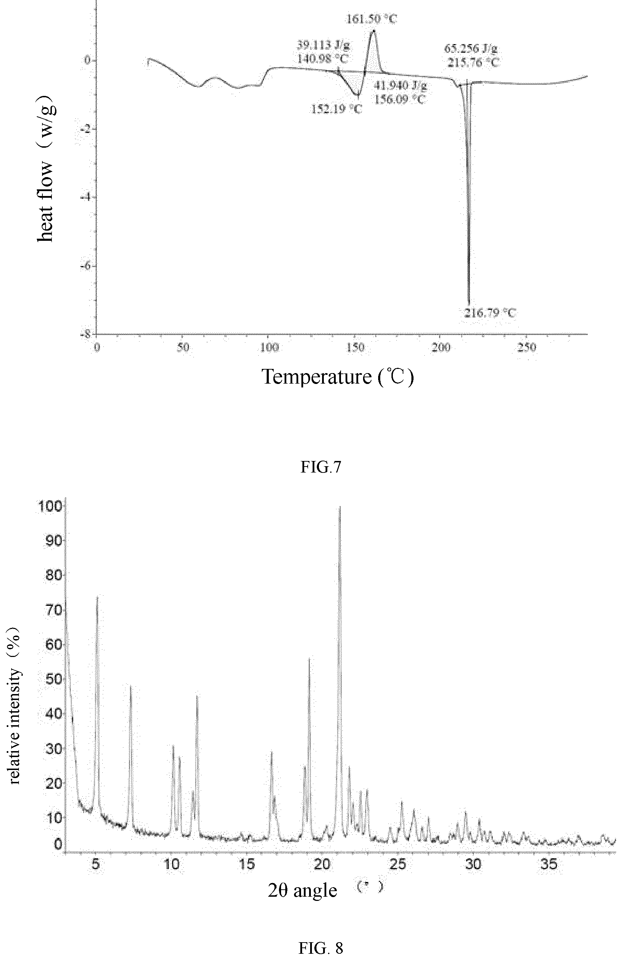

In the present application, as one of the embodiments, the XRPD diagram of the crystal form B of the compound of formula (II) is shown in FIG. 8.

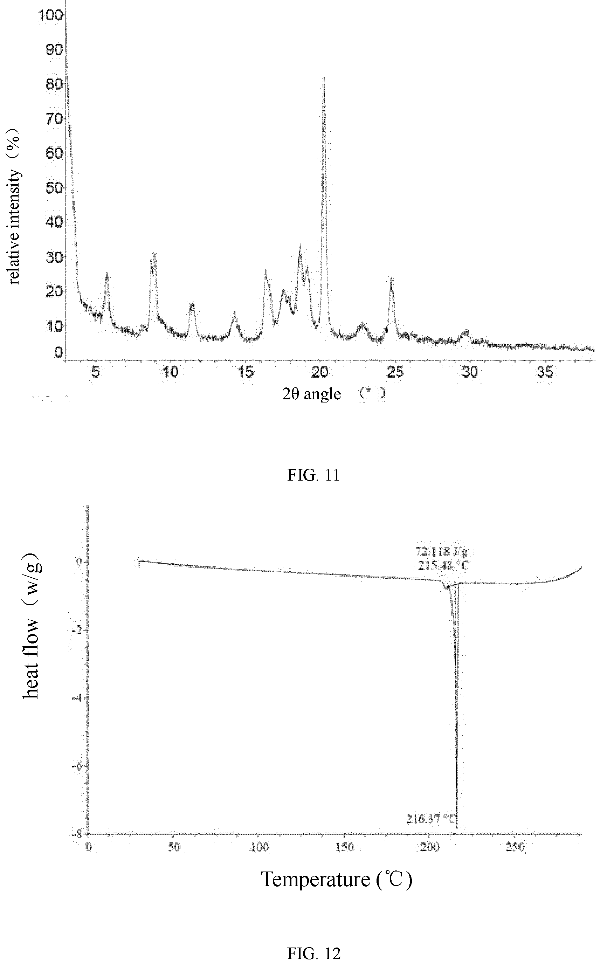

In the present application, as one of the embodiments, the XRPD diagram of the crystal form C of the compound of formula (II) is shown in FIG. 11.

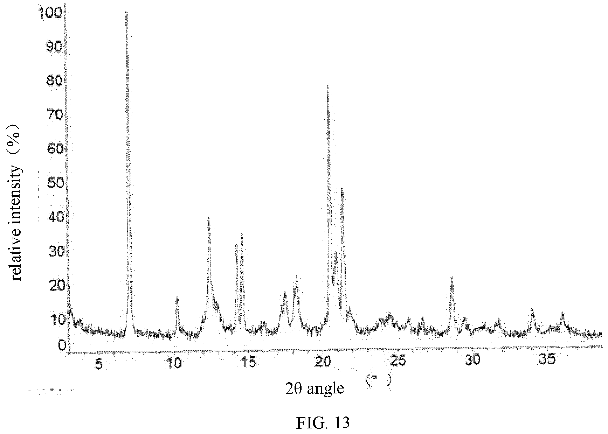

In the present application, as one of the embodiments, the XRPD diagram of the crystal form D of the compound of formula (II) is shown in FIG. 13.

In the oral preparation provided in the present application, the pharmaceutically acceptable salt of the active ingredient can be selected from a group consisting of (S)-N-(5- (2-(2,2-difluorocyclopropane carbonyl)-2-azaspiro[3.5]non 7-yl)-[1,2,4]triazole [1,5-a] pyridin-2-yl) cyclopropane formamide or pharmaceutically acceptable salt thereof, with an active ingredient content of 1% to 50%, preferably 5% to 40%, further preferably 6% to 30%, more preferably 8% to 25% of the total weight of the composition. As an exemplary illustration, it can be 10%, 11%, 12%, 13%, 14%, 15%, 16%, 17%, 18%, 19%, or 20%.

The oral preparation provided in the present application may further include one or more of the fillers, disintegrants, adhesives, lubricants, and surfactants.

In the oral preparation provided in the present application, the filler can include, but not limited to, lactose, microcrystalline cellulose, pre-gelatinized starch, anhydrous calcium hydrogen phosphate, calcium sulfate, calcium carbonate, dextrin, maltose, mannitol, sorbitol, trehalose, xylitol, etc. In a preferred embodiment of the present application, the filler is one or more selected from a group consisting of lactose, microcrystalline cellulose, pre-gelatinized starch, and anhydrous dicalcium phosphate. In a more preferred embodiment, the filler is a mixture of lactose and microcrystalline cellulose.

In the oral preparation provided in the present application, the content of the filler can be 0% to 78.5%, preferably 10% to 70%, further preferably 20% to 60%, more preferably 30% to 50% of the total weight of the pharmaceutical composition. As an exemplary illustration, it can be 30%, 31.75%, 37.25%, 39.25%, 41.75%, 42.25%, 43.75%. The weight ratio of lactose to microcrystalline cellulose is 0:1, 5:3, 3:1, 1:1, 1:0; and preferably 1:1.

In the oral preparation provided in the present application, disintegrating agents may include, but not limited to, cross-linked carboxymethyl cellulose sodium, carboxymethyl starch sodium, cross-linked polyvinylketone, dry starch, low substituted hydroxypropyl cellulose, alginate, chitosan, and corn starch. In a preferred embodiment of the present application, the disintegrant in the composition may include, but not limited to, one or more cross-linked carboxymethyl cellulose sodium, carboxymethyl starch sodium, or cross-linked polyvinone. In a preferred embodiment of the present application, the disintegrant is cross-linked carboxymethyl cellulose sodium.

The content of the disintegrating agent in the oral preparation provided in the present application can be 1% to 25%, preferably 2% to 16%, more preferably 4%, 8%, or 12%, of the total weight of the pharmaceutical composition.

In the oral preparation provided in the present application, the adhesive may include, but not limited to, to one or more hydroxypropyl methylcellulose, hydroxypropyl cellulose, polyvinylketone, pre-gelatinized starch, sodium carboxymethyl cellulose, and methyl cellulose.

In the oral preparation provided in the present application, the content of the adhesive, based on the total weight of the composition, is about 0.1% to 5%, preferably 1% to 4%, more preferably 2% to 3%, and most preferably 1.5% (20 mg composition for example, which contains 3 mg of adhesive).

The oral preparation provided in the present application may further contain one or more lubricants, such as magnesium stearate, micronized silica gel, talc powder, hydrogenated vegetable oil, polyethylene glycol, and sodium dodecyl sulfate, with a lubricant content of 0.1% to 5%, preferably 0.5% to 3%, and more preferably 1% of the total weight of the composition.

The oral preparation provided by the present application may further contain one or more surfactants, such as sodium dodecyl sulfate (SDS), with a content of approximately 0.1% to 5%; preferably 1% to 3%; most preferably 2%, based on the total weight of the composition.

In a preferred embodiment of the present application, a pharmaceutical composition is provided, including the following components by weight:
1) 1% to 50% (S)-N-(5- (2- (2,2-difluorocyclopropane carbonyl)-2-azaspiro[3.5]non 7-yl)-[1,2,4]triazole [1,5-a] pyridin-2-yl) cyclopropane formamide or pharmaceutically acceptable salts thereof;
2) 0%~78.5% filler, being one or two selected from lactose and microcrystalline cellulose;
3) 1% -25% disintegrating agent, being one or more selected from a cross-linked carboxymethyl cellulose sodium, carboxymethyl starch sodium, cross-linked polyvinylketone;
4) 0.1%~5% adhesive, being one or more selected from hydroxypropyl methylcellulose, hydroxypropyl cellulose, or polyvinylketone;
5) 0.1%~5% lubricant, being magnesium stearate; and
6) 0.1%~5% surfactant, being sodium dodecyl sulfate (SDS).

In another preferred embodiment of the present application, a pharmaceutical composition is provided, including the following components by weight:
1) 8% -25% (S)-N-(5- (2- (2,2-difluorocyclopropane carbonyl)-2-azaspiro[3.5]non 7-yl)-[1,2,4]triazole [1,5-a] pyridin-2-yl) cyclopropane formamide or pharmaceutically acceptable salts thereof;
2) 30-50% filler, being one or two of selected from lactose and microcrystalline cellulose;
3) 2%~16% disintegrating agent, being one or more selected from cross-linked carboxymethyl cellulose sodium, carboxymethyl starch sodium, cross-linked polyvinylketone;
4) 1% -4% adhesive, being one or more selected from a group consisting of hydroxypropyl methyl cellulose, hydroxypropyl cellulose, polyvinylketone;
5) 0.5% -3% lubricant, being magnesium stearate; and
6) 1% -3% surfactant, being sodium dodecyl sulfate (SDS).

The oral preparation of the present application can be prepared using commonly used preparation methods in the field, for example, preparing pharmaceutical composition granules by wet granulation, dry granulation, one-step granulation, and the like, and then tableting. The pharmaceutical composition of the present application can be further prepared into tablets by powder direct pressing method. In the present application, as one of the embodiments, the filler, disintegrant, adhesive, lubricant or surfactant can be added internally or externally. As an exemplary explanation, the filler can be added internally or externally. The disintegrant can be added internally or externally. The adhesive can be added internally or externally, and the lubricant can be added internally or externally. The surfactant is added internally or externally.

The oral preparation of the present application dissolves very quickly and completely. According to the second method (paddle method) of General Rule 0931 in Part IV of the Chinese Pharmacopoeia 2015 edition, 0.01mol/L hydrochloric acid solution is used as the dissolution medium, preferably 900ml of 0.01mol/L hydrochloric acid solution. The dissolution test of the combination of the present application is carried out at a paddle speed of 50rpm at 37 ± 0.5 °C, with a dissolution rate of 80% or more in 10 minutes or 15 minutes, preferably a dissolution rate of 90% or more in 15 minutes or 20 minutes, and more preferably a dissolution rate be greater than or equal to 95% in 30 minutes or 45 minutes.

The oral preparation of the present application is placed in a watch glass and subjected to stability tests under high temperature (60 °C), high humidity (relative humidity 75% ± 1%, 15.5-60 °C), and light (illumination 4500lx ± 500lx) conditions. Samples are taken at 5, 10, and 30 days respectively, and changes in relative substances, content, and dissolution rate are measured using HPLC. The results show that the composition provided by the present application has stable properties, and as are compared 5, 10, 30 days with 0 days, there were no significant differences of influencing factors like the relevant substances, dissolution, and content.

The oral preparation of the present application was placed in an environment with a temperature of 40 °C± 2 °C and a relative humidity of 75% ± 5% for stability evaluation. After being placed for one month, the changes in the relevant substances, content, and dissolution were determined using HPLC method. The results showed that the composition provided by the present application had stable properties, and as compared acceleration for one month with 0 days, there was no significant difference in the relevant substances, dissolution, and content.

### BRIEF DESCRIPTION OF THE DRAWINGS

FIG. 1 shows the dissolution curves of the tablets of Examples 1-7 in 0.01mol/L hydrochloric acid solution.
FIG. 2 shows the dissolution curves of the tablets of Examples 1, 8, and 9 in 0.01mol/L hydrochloric acid solution.
FIG. 3 shows the dissolution curves of the tablets of Example 1, 10-13 in 0.01mol/L hydrochloric acid solution.
FIG. 4 shows the dissolution curves of the tablets from Examples 1, 14 to 18 in 0.01mol/L hydrochloric acid solution.
FIG. 5 shows the dissolution curves of the tablets of Examples 1 and 19 in 0.01mol/L hydrochloric acid solution.
FIG. 6: XRPD diagram of crystal form A.
FIG. 7: DSC spectrum of crystal form A.
FIG. 8: XRPD diagram of crystal form B.
FIG. 9: DSC diagram of crystal form B.
FIG. 10: TGA spectrum of crystal form B.
FIG. 11: XRPD diagram of crystal form C.
FIG. 12: DSC diagram of crystal form C.
FIG. 13: XRPD diagram of crystal form D.

### DEFINITION AND DESCRIPTION

Unless otherwise specified, the following terms and phrases used in this article are intended to have the following meanings. A specific term or phrase should not be considered uncertain or unclear without a specific definition, but should be understood according to its ordinary meaning. When a product name is mentioned herein, it refers to its corresponding product or its active ingredient.

The term "pharmaceutically acceptable" used in the present application refers to compounds, materials, compositions, and/or dosage forms that are within the scope of reliable medical judgment and are suitable for use in contact with human and animal tissues, without excessive toxicity, irritation, allergic reactions, or other issues or complications, and are proportional to a reasonable benefit/risk ratio.

The term "pharmaceutically acceptable salt" refers to the salt of a compound of the present application, prepared by the discovery of a compound with a specific substituent group and a relatively non-toxic acid or base. When the compound of the present application contains relatively acidic functional groups, alkali addition salts can be obtained by contacting the neutral form of such compounds with a sufficient amount of alkali in a pure solution or suitable inert solvent. Pharmaceutically acceptable alkali addition salts include sodium, potassium, calcium, ammonium, organic amine or magnesium salts, or similar salts. When the compound of the present application contains relatively basic functional groups, acid addition salts can be obtained by contacting the neutral form of such compounds with a sufficient amount of acid in a pure solution or suitable inert solvent. Examples of pharmaceutically acceptable acid addition salts include inorganic acid salts, which include, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphite, etc; And organic acid salts, including acids such as acetic acid, propionic acid, isobutyric acid, maleic acid, malonic acid, benzoic acid, succinic acid, succinic acid, suberic acid, fumaric acid, lactic acid, mandelic acid, phthalic acid, benzenesulfonic acid, p-toluenesulfonic acid, citric acid, tartaric acid, and methanesulfonic acid; It further includes salts of amino acids such as arginine, as well as salts of organic acids such as glucuronic acid. Some specific compounds of the present application contain basic and acidic functional groups, which can be converted into any base or acid addition salt.

The "pharmaceutical composition" described in the present application is typically used for oral administration. The pharmaceutical composition used for oral administration may further include sweeteners, flavouring, colorants, coating agents, and/or preservatives to provide palatable formulations. In one embodiment, the pharmaceutical composition is in the form of tablets. Tablets can be prepared by pressing or molding. Compressed tablets can be prepared by pressing free flowing active ingredients such as powders or particles in a suitable machine, optionally mixed with adhesives, lubricants, inert diluents, or preservatives. Molded tablets can be prepared by molding in a suitable machine and wetting a mixture of powdered active ingredients with an inert liquid diluent. Tablets can be optionally coated or scored.

The "pharmaceutical preparations" referred to in the present application refers to a method of combining different chemical substances (including active drugs) to produce the final medicinal product. Pharmaceutical preparations include enteral preparations (tablets, capsules), parenteral preparations (liquids, freeze-dried powders), or topical preparations (skin, inhalable).

The "pharmaceutically acceptable excipients, carriers, or diluents" of the present application include, but not limited to, any adjuvants, carriers, excipients, retention aids, additives, diluents, preservatives, dyes/colorants, flavoring agents, surfactants, wetting agents, dispersants, suspension aids, stabilizers, isotopes, solvents or emulsifiers licensed by relevant government regulatory authorities as acceptable for human or livestock use.

The term "filler" refers to a substance that can improve the compressibility and uniformity of a drug. Fillers include starch, sucrose, dextrin, lactose, pre-gelatinized starch, microcrystalline cellulose, corn starch, dextrose, ethyl cellulose, fructose, maltodextrin, maltose, medium chain triglycerides, anhydrous calcium hydrogen phosphate, calcium sulfate, calcium carbonate, sugar alcohol erythritol, isomaltosol, lactitol, mannitol, sorbitol, trehalose, and xylitol.

The term "disintegrating agent" refers to an excipient that causes a tablet to rapidly break into small particles in gastrointestinal fluid, mainly used to eliminate the binding force generated by adhesion and/or high compression, thereby causing the tablet to disintegrate in water. Disintegration agents include dry starch, sodium carboxymethyl starch, low substituted hydroxypropyl cellulose, cross-linked sodium carboxymethyl cellulose, cross-linked povidone, microcrystalline cellulose, alginate, sodium alginate, etc.

The term "adhesive" refers to an auxiliary material that has inherent viscosity and can impart appropriate viscosity to materials without viscosity or with insufficient viscosity, promoting the bonding of solid powder into larger particles, which helps to make stronger dosage forms. Binders include starch slurry, cellulose derivatives, methyl cellulose, hydroxypropyl cellulose, hydroxypropyl methylcellulose, sodium carboxymethyl cellulose, povidone, gelatin, 50%~70% sucrose solution, sodium alginate solution, etc.

The term "lubricant" refers to the material that prevents material from accumulating and adhering to the surface of the punch and punch, or adhering to the capsule filling machine. Lubricants can improve the surface characteristics of particles, such as improving the electrostatic distribution on the particle surface, improving the roughness of the particle surface, reducing friction, improving gas selective adsorption, and weakening the van der Waals force between particles. Lubricants include magnesium stearate, micropowder silica gel, talc, hydrogenated vegetable oil, polyethylene glycols, sodium dodecyl sulfate, hydrogenated castor oil, cottonseed oil, behenic acid glyceride, monostearin glyceride, palmitic acid glyceride, medium chain triglyceride, mineral oil, light mineral oil, octyl lauryl alcohol, poloxamer, polyethylene glycol, polyoxyethylene stearate, polyvinyl alcohol, etc.

The autoimmune diseases mentioned in the present application refer to rheumatoid arthritis, inflammatory bowel disease (ulcerative colitis, Krohn's disease), systemic lupus erythematosus, dermatomyositis, ankylosing spondylitis, multiple sclerosis, type I diabetes, psoriasis, vitiligo, Sjogren's syndrome, etc., or other inflammatory skin diseases such as atopic dermatitis, eczema, lichen planus, lichen lustre, lichen scleroatrophicus, panniculitis, acne Purulent sweat gland inflammation, etc.

### DETAILED DESCRIPTION

The present application is further explained in detail through the following embodiments. These embodiments are provided for illustrative purposes only and are not intended to limit the scope of the present application.

### Examples 1-7

Crushed TUL01101, lactose, microcrystalline cellulose, cross-linked carboxymethyl cellulose sodium, carboxymethyl starch sodium, cross-linked polyvinylketone, and substituted hydroxypropyl cellulose were subjected to wet granulation using an efficient wet mixing granulator according to the ratio in Table 1. 3.4% hydroxypropyl methylcellulose and 2.3% sodium dodecyl sulfate aqueous solution were used as a granulation solution. After granulation, wet granulation and drying treatment were carried out, and then dried particles (moisture content<3%) were dry granulated, added externally with cross-linked sodium carboxymethyl cellulose or sodium carboxymethyl starch, cross-linked polyvinylketone, low substituted hydroxypropyl cellulose, and magnesium stearate, mixed well, and tableted.

**Table 1**

| Ingredients | Example (mg/tablet) | | | | | | |
|---|---|---|---|---|---|---|---|
| | 1 | 2 | 3 | 4 | 5 | 6 | 7 |
| TUL01101 | 20.0 | 20.0 | 20.0 | 20.0 | 20.0 | 20.0 | 20.0 |
| Lactose | 78.5 | 78.5 | 78.5 | 84.5 | 74.5 | 84.5 | 78.5 |
| Microcrystalline cellulose | 78.5 | 78.5 | 78.5 | 84.5 | 74.5 | 84.5 | 78.5 |
| Cross linked carboxymethyl cellulose sodium (added internally) | 8.0 | - | - | 2.0 | 12.0 | - | - |
| Sodium carboxymethyl starch (added internally) | - | 8.0 | - | - | - | - | - |
| Crosslinked polyvinylketone (added internally) | - | - | 8.0 | - | - | - | - |
| Low substituted hydroxypropyl cellulose (added internally) | - | - | - | - | - | 2.0 | 8.0 |
| Hydroxypropyl methylcellulose | 3.0 | 3.0 | 3.0 | 3.0 | 3.0 | 3.0 | 3.0 |
| Sodium dodecyl sulfate | 2.0 | 2.0 | 2.0 | 2.0 | 2.0 | 2.0 | 2.0 |
| Cross linked carboxymethyl cellulose sodium (added externally) | 8.0 | - | - | 2.0 | 12.0 | - | - |
| Sodium carboxymethyl starch (added externally) | - | 8.0 | - | - | - | - | - |
| Crosslinked polyvinylketone (added externally) | - | - | 8.0 | - | - | - | - |
| Low substituted hydroxypropyl cellulose (added internally) | - | - | - | - | - | 2.0 | 8.0 |
| Magnesium stearate | 2.0 | 2.0 | 2.0 | 2.0 | 2.0 | 2.0 | 2.0 |
| Tablet weight | 200 | 200 | 200 | 200 | 200 | 200 | 200 |

### Experiment Example 1 Dissolution Experiment

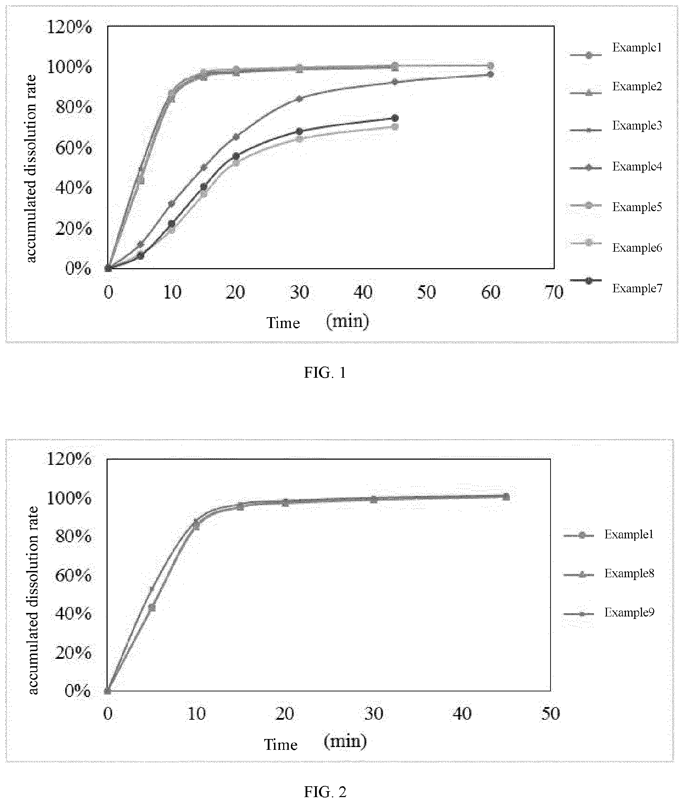

According to the second method (paddle method) of General Rule 0931 in Part 4 of the Chinese Pharmacopoeia 2020, the dissolution of the tablets in Examples 1-7 was determined. 900ml 0.01mol/L hydrochloric acid solution was used as the dissolution medium and a dissolution test was conducted at a propeller speed of 50 rpm at 37 ± 0.5 °C. The results showed that the compound TLTLO1101 in Examples 4, 6, and 7 had a slow dissolution rate and did not fully dissolve after 45 minutes. The compound TUL01101 in the other embodiments fully dissolved and had similar dissolution behavior. Based on the research results of the above embodiments, cross-linked carboxymethyl cellulose sodium, carboxymethyl starch sodium, and cross-linked polyvinylketone are preferred as disintegrants. As TUL01101 is sensitive to moisture, cross-linked carboxymethyl cellulose sodium with relatively low moisture absorption is the most preferred disintegrant. Since increasing the dosage of disintegrant does not significantly improve the dissolution behavior, the optimal dosage of disintegrant is 8.0%. The dissolution data is shown in Table 2, and the dissolution curve is shown in FIG. 1.

**Table 2**

| Time (min) | Dissolution (%) | | | | | | |
|---|---|---|---|---|---|---|---|
| | 1 | 2 | 3 | 4 | 5 | 6 | 7 |
| 5 | 43.4 | 43.6 | 49.2 | 11.9 | 44.5 | 7.1 | 6.2 |
| 10 | 85.6 | 84.2 | 87.5 | 32.3 | 87.0 | 19.2 | 22.3 |
| 15 | 95.3 | 95.0 | 96.2 | 50.1 | 97.0 | 36.8 | 40.6 |
| 20 | 97.6 | 97.0 | 97.6 | 65.2 | 98.7 | 52.3 | 55.7 |
| 30 | 99.1 | 98.6 | 99.5 | 84.2 | 99.5 | 64.2 | 67.9 |
| 45 | 100.5 | 99.6 | 100.2 | 92.3 | 100.4 | 70.3 | 74.6 |
| 60 | - | - | - | 96.1 | 100.6 | - | - |

### Experiment Example 2: Stability study

### (1) Influencing factor test

The tablet of Example 1 was placed in a watch glass and conduct stability tests under high temperature (60 °C), high humidity (relative humidity 75% ± 1%, 15.5-60 °C), and light (illumination 4500lx ± 500lx) conditions. Samples were taken at 5 days, 10 days, and 30 days, respectively. The changes in related substances, content, and dissolution were determined using HPLC method. The results showed that the tablet of Example 1 had stable properties, and there was no significant difference of the influencing factors such as relevant substances, dissolution and content at 5th day, 10th day, and 30th day as compared with 0 day, data is shown in Table 3.

**Table 3**

| Test items | 0 days | High temperature | | | High humidity | | | Illumination | | |
|---|---|---|---|---|---|---|---|---|---|---|
| | | 5 days | 10 days | 30 days | 5 days | 10 days | 30 days | 5 days | 10 days | 30 days |
| Related substances (total impurities) | 0.3% | 0.3% | 0.3% | 0.3% | 0.3% | 0.3% | 0.3% | 0.3% | 0.3% | 0.3% |
| Dissolution (30min) | 99.1 % | 95.2% | 93.1% | 92.5% | 96.7% | 91.1 % | 93.7% | 94.4% | 92.4 % | 92.3% |
| Content determinati on | 99.7 % | 100.2 % | 100.4 % | 100.0 % | 101.7 % | 99.6 % | 100.9 % | 101.5 % | 98.5 % | 100.0 % |

### (2) Accelerated testing

The table of Example 1 was placed in an environment with a temperature of 40 °C± 2 °C and a relative humidity of 75% ± 5% for stability evaluation. After being placed for one month, the changes of related substances, content, and dissolution were determined by HPLC method. The results showed that the tablet of Example 1 had stable properties, and the results were accelerated for one month compared to 0 day. There were no significant differences in relevant substances, dissolution, and content, data is shown in Table 4.

**Table 4**

| Test items | 0 month | 1 month |
|---|---|---|
| Related substances (total impurities) | 0.3% | 0.3% |
| Dissolution (30min) | 99.1% | 94.9% |
| Content determination | 99.7% | 100.0% |

### Examples 8-9

After grinding treatment, the Crushed TUL01101, lactose, microcrystalline cellulose, and cross-linked carboxymethyl cellulose sodium were subjected to wet granulation using an efficient wet mixing granulator according to the ratio in Table 5. 3.4% hydroxypropyl methyl cellulose or hydroxypropyl cellulose, polyvinylketone, and 2.3% sodium dodecyl sulfate aqueous solution were used as the granulation solution. After granulation, wet granulation and drying treatment were carried out, and then the dried particles (moisture<3%) were size prepared under a dry condition, added externally with cross-linked sodium carboxymethyl cellulose and magnesium stearate, mixed well, and then tableted.

**Table 5**

| Ingredients | Examples (mg/tablet) | | |
|---|---|---|---|
| | 1 | 8 | 9 |
| TUL01101 | 20.0 | 20.0 | 20.0 |
| Lactose | 78.5 | 78.5 | 78.5 |
| Microcrystalline cellulose | 78.5 | 78.5 | 78.5 |
| Cross linked carboxymethyl cellulose sodium (added internally) | 8.0 | 8.0 | 8.0 |
| Hydroxypropyl methylcellulose | 3.0 | - | - |
| Hydroxypropyl cellulose | - | 3.0 | - |
| Povidone | - | - | 3.0 |
| Sodium dodecyl sulfate | 2.0 | 2.0 | 2.0 |
| Cross linked carboxymethyl cellulose sodium (added externally) | 8.0 | 8.0 | 8.0 |
| Magnesium stearate | 2.0 | 2.0 | 2.0 |
| tablet weight | 200 | 200 | 200 |

### Experiment Example 3 Dissolution Experiment

According to the second method (paddle method) of General Rule 0931 in Part 4 of the Chinese Pharmacopoeia 2020, the dissolution of the tablets in Examples 1 and 8-9 was determined. 900ml 0.01mol/L hydrochloric acid solution was used as the dissolution medium and a dissolution test was conducted at a propeller speed of 50rpm at 37 ± 0.5 °C. The results showed that in Example 1, Example 8, and Example 9, TUL01101 completely dissolved and had similar dissolution behavior. Based on the research results of the above embodiments, the type of adhesives has no significant impact on the dissolution behavior of the formulation. The dissolution data is shown in Table 6, and the dissolution curve is shown in FIG. 2.

**Table 6**

| Time (min) | Dissolution (%) | | |
|---|---|---|---|
| | 1 | 8 | 9 |
| 5 | 43.4 | 42.8 | 52.6 |
| 10 | 85.6 | 84.8 | 88.0 |
| 15 | 95.3 | 95.1 | 96.7 |
| 20 | 97.6 | 97.1 | 98.3 |
| 30 | 99.1 | 98.8 | 100.0 |
| 45 | 100.5 | 100.5 | 101.1 |

### Examples 10-13

After grinding treatment, the crushed TUL01101, lactose, microcrystalline cellulose, pre-gelatinized starch, anhydrous dicalcium phosphate, and cross-linked carboxymethyl cellulose sodium were subjected to wet granulation using an efficient wet mixing granulator according to the ratio in Table 7. 3.4% hydroxypropyl methyl cellulose and 2.3% sodium dodecyl sulfate aqueous solution were used as granulation solution. After granulation, wet granulation and drying treatment are carried out, and then the dried particles (moisture<3%) were subjected to dry size preparation, added externally with cross-linked sodium carboxymethyl cellulose and magnesium stearate, mixed well, and then tableted.

**Table 7**

| Ingredients | Example (mg/tablet) | | | | |
|---|---|---|---|---|---|
| | 1 | 10 | 11 | 12 | 13 |
| TUL01101 | 20.0 | 20.0 | 20.0 | 20.0 | 20.0 |
| lactose | 78.5 | 0 | - | - | 157.0 |
| Anhydrous calcium hydrogen phosphate | - | - | 78.5 | - | - |
| pre-gelatinized starch | - | - | - | 78.5 | - |
| Microcrystalline cellulose | 78.5 | 157.0 | 78.5 | 78.5 | 0 |
| Cross linked carboxymethyl cellulose sodium (added internally) | 8.0 | 8.0 | 8.0 | 8.0 | 8.0 |
| Hydroxypropyl methylcellulose | 3.0 | 3.0 | 3.0 | 3.0 | 3.0 |
| Sodium dodecyl sulfate | 2.0 | 2.0 | 2.0 | 2.0 | 2.0 |
| Cross linked carboxymethyl cellulose sodium (added externally) | 8.0 | 8.0 | 8.0 | 8.0 | 8.0 |
| Magnesium stearate | 2.0 | 2.0 | 2.0 | 2.0 | 2.0 |
| tablet weight | 200 | 200 | 200 | 200 | 200 |

### Experiment Example 4 Dissolution Experiment

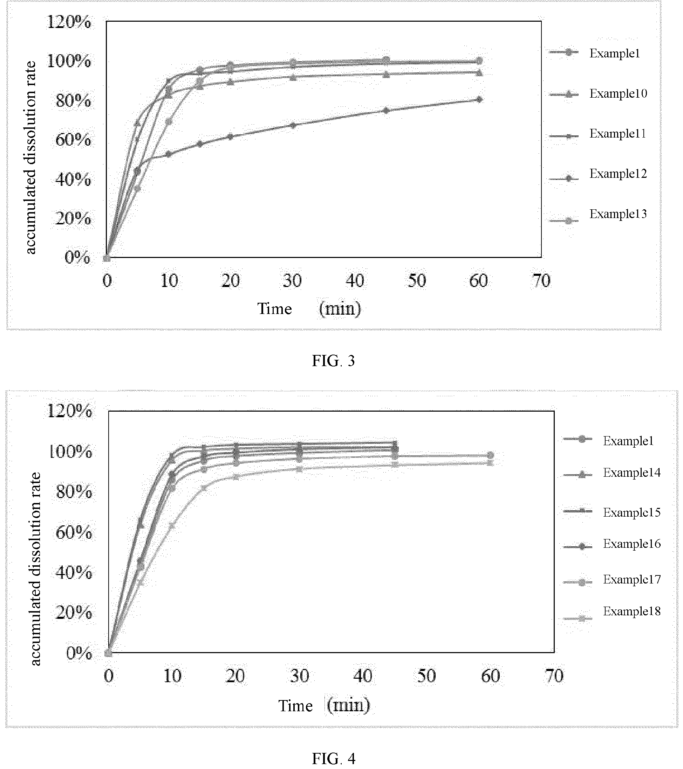

According to the second method (paddle method) of General Rule 0931 in Part 4 of the Chinese Pharmacopoeia 2020, the dissolution of the tablets in Examples 1 and 10-13 was determined. 900ml 0.01mol/L hydrochloric acid solution was used as the dissolution medium and a dissolution test was conducted at a propeller speed of 50rpm at 37 ± 0.5 °C. The results showed that in Example 12, compound TUL01101 slowly dissolved and could not completely dissolved, while in other embodiments, compound TUL01101 completely dissolved. Based on the research results of the above embodiments, the type and dosage of fillers will affect the dissolution behavior of the formulation. The dissolution data is shown in Table 8, and the dissolution curve is shown in FIG. 3.

**Table 8**

| Time (min) | Dissolution (%) | | | | |
|---|---|---|---|---|---|
| | 1 | 10 | 11 | 12 | 13 |
| 5 | 43.4 | 68.7 | 59.9 | 44.7 | 35.2 |
| 10 | 85.6 | 82.6 | 89.5 | 52.3 | 68.8 |
| 15 | 95.3 | 87.0 | 93.3 | 57.5 | 89.6 |
| 20 | 97.6 | 89.2 | 94.4 | 61.3 | 96.5 |
| 30 | 99.1 | 91.8 | 96.8 | 67.2 | 98.5 |
| 45 | 100.5 | 93.2 | 98.5 | 74.6 | 99.4 |
| 60 | - | 94.1 | 99.2 | 80.1 | 99.9 |

### Examples 14-18

After grinding treatment, the crushed TUL01101, lactose, microcrystalline cellulose, and cross-linked carboxymethyl cellulose sodium were subjected to wet granulation using an efficient wet mixing granulator according to the ratio in Table 9. 3.4% hydroxypropyl methyl cellulose and 2.3% sodium dodecyl sulfate aqueous solution were used as the granulation solution. After granulation, wet granulation and drying treatment were carried out. Then, the dried particles (with a moisture content of less than 3%) were subjected dry size preparation, added externally with cross-linked carboxymethyl cellulose sodium and magnesium stearate, mixed well and tableted.

**Table 9**

| Ingredients | Example (mg/tablet) | | | | | |
|---|---|---|---|---|---|---|
| | 1 | 14 | 15 | 16 | 17 | 18 |
| TUL01101 | 20.0 | 1.0 | 5.0 | 10.0 | 50.0 | 100.0 |
| lactose | 78.5 | 43.75 | 41.75 | 39.25 | 63.5 | 127.0 |
| Microcrystalline cellulose | 78.5 | 43.75 | 41.75 | 39.25 | 63.5 | 127.0 |
| Cross linked carboxymethyl cellulose sodium(added internally) | 8.0 | 4.0 | 4.0 | 4.0 | 8.0 | 16.0 |
| Hydroxypropyl methylcellulose | 3.0 | 1.5 | 1.5 | 1.5 | 3.0 | 6.0 |
| Sodium dodecyl sulfate | 2.0 | 1.0 | 1.0 | 1.0 | 2.0 | 4.0 |
| Cross linked carboxymethyl cellulose sodium (added externally) | 8.0 | 4.0 | 4.0 | 4.0 | 8.0 | 16.0 |
| Magnesium stearate | 2.0 | 1.0 | 1.0 | 1.0 | 2.0 | 4.0 |
| tablet weight | 200 | 100 | 100 | 100 | 200 | 400 |

### Experiment Example 5 Dissolution Experiment

According to the second method (paddle method) of General Rule 0931 in Part 4 of the Chinese Pharmacopoeia 2020, the dissolution of the tablets in Examples 1 and 14-18 was determined. 900ml 0.01mol/L hydrochloric acid solution was used as the dissolution medium and a dissolution test was conducted at a propeller speed of 50rpm at 37 ± 0.5 °C. The results showed that in Example 18, compound TUL01101 had a slow dissolution, due to the excessive content of the main drug in a single dose of the formulation in Example 18, resulting in a slower dissolution behavior due to the limitation of the solubility of the main drug. The dissolution behavior of other specifications of the formulation was similar. The dissolution data is shown in Table 10, and the dissolution curve is shown in FIG. 4.

**Table 10**

| Time (min) | Dissolution (%) | | | | | |
|---|---|---|---|---|---|---|
| | 1 | 14 | 15 | 16 | 17 | 18 |
| 5 | 43.4 | 63.7 | 66.1 | 45.7 | 42.4 | 34.7 |
| 10 | 85.6 | 95.6 | 98.1 | 88.5 | 81.5 | 62.9 |
| 15 | 95.3 | 100.2 | 102.0 | 97.4 | 91.1 | 81.6 |
| 20 | 97.6 | 101.1 | 103.1 | 99.2 | 94.0 | 87.3 |
| 30 | 99.1 | 101.9 | 103.6 | 100.9 | 96.2 | 91.1 |
| 45 | 100.5 | 102.0 | 104.3 | 101.8 | 97.5 | 93.0 |
| 60 | - | - | - | - | 97.8 | 94.0 |

### Example 19

After grinding treatment, rushed TUTL01101, lactose, microcrystalline cellulose, sodium dodecyl sulfate, cross-linked carboxymethyl cellulose sodium, and magnesium stearate were well mixed in the proportions shown in Table 11, then tableted directly.

**Table 11**

| Ingredients | Example (mg/tablet) |
|---|---|
| | 19 |
| TUL01101 | 20.0 |
| Lactose | 100.0 |
| Microcrystalline cellulose | 60.0 |
| Sodium dodecyl sulfate | 2.0 |
| Cross linked carboxymethyl cellulose sodium | 16.0 |
| Magnesium stearate | 2.0 |
| Sheet weight | 200 |

### Experiment Example 6 Dissolution Experiment

According to the second method (paddle method) of General Rule 0931 in Part 4 of the Chinese Pharmacopoeia 2020, the dissolution of the tablets in Example 1 and Example 19 was determined. Use 900ml 0.01mol/L hydrochloric acid solution as the dissolution medium and conduct a dissolution test at a propeller speed of 50rpm at 37 ± 0.5 °C. The dissolution data is shown in Table 12, and the dissolution curve is shown in FIG. 5. The results showed that in Example 19, compound TUL01101 was slowly dissolved and could not be completely dissolved. Based on the research results of the above embodiments, the wet granulation process is preferred.

**Table 12**

| Time (min) | Dissolution (%) | |
|---|---|---|
| | 1 | 19 |
| 5 | 43.4 | 59.8 |
| 10 | 85.6 | 76.8 |
| 15 | 95.3 | 82.4 |
| 20 | 97.6 | 84.5 |
| 30 | 99.1 | 86.4 |
| 45 | 100.5 | 88.4 |
| 60 | - | 88.7 |

### Example 20 Preparation of Compound of Formula (II)

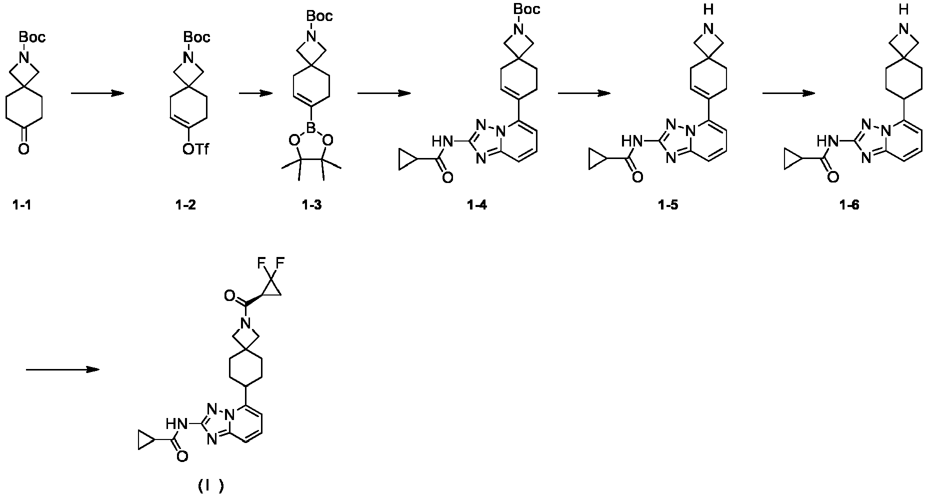

Step 1: LiHMDS (1 M, 51.2 mL) was added dropwise to a THF (150 mL) solution containing compound 1-1 (10.2 g, 42.6 mmol) at -78 °C. The reaction solution was stirred at -78 °C for 1 hour, then THF (150 mL) solution of 1,1,1-trifluoro-N-phenyl-N-(trifluoromethyl sulfonyl group) methanesulfonamide (16.7 g, 46.9 mmol) was added to the reaction solution, and then stirred at 15 °C for 12 hours. The reaction was quenched with 250 mL of saturated ammonium chloride, and diluted with 200 mL of water, and then extracted with ethyl acetate (200 mL * 3). The organic phases were combined, washed with saturated salt water, dried with sodium sulfate, filtered and concentrated to obtain Compound 1-2. The crude product was directly used for the next reaction without purification.

1H NMR (400 MHz, CDCl3) δ 5.63 (br s, 1H) ,3.50-3.65 (m, 4H), 2.34 (br s, 4H), 1.88 (br t, J=5.90 Hz, 2H), 1.37 (s, 9H)

Step 2: potassium acetate (12.7 g, 129.3 mmol) and Pd (dppf) Cl2.CH2Cl2 (3.5 g, 4.3 mmol) were added to a DMF (100 mL) solution containing Compound 1-2 (16 g, 43.1 mmol) and pinacol diborate (12.0 g, 47.4 mmol). The solution was replaced with nitrogen three times and kept stirring at 70 ° C for 3 hours in a nitrogen atmosphere. The reaction solution was dispersed in a mixture of 300 mL of water and 400 mL of ethyl acetate. The organic phases were separated and washed with saturated salt water, dried with sodium sulfate, filtered and concentrated to obtain the crude product. The crude product was purified by silica gel chromatography to obtain Compound 1-3.

1H NMR (400MHz, CDCl₃) δ 6.46 (br s, 1H), 3.71 - 3.53 (m, 4H), 2.31 (br d, J=3.0 Hz, 2H), 2.24 - 2.16 (m, 2H), 1.74 (t, J=6.3 Hz, 2H), 1.44 (s, 9H), 1.26 (s, 12H)

Step 3: In a nitrogen atmosphere, potassium carbonate (3.8 g, 27.3 mmol) and Pd (dppf) Cl2.CH2Cl2 (744 mg, 911.0 µmol) were added to a solution of Compound 1-3 (3.5 g, 10.0 mmol) and N-(5-bromo-[1,2,4]triazolo[1,5-a]pyridin-2-yl)cyclopropaneformamide (2.6 g, 9.1 mmol) dissolved in dioxane (60 mL) and water (15 mL). The reaction solution was stirred at 90 °C for 3 hours. The reaction solution was concentrated to obtain a crude product, which was separated and purified to obtain Compound 1-4 through column chromatography. LCMS (ESI) m/z: 324.1[M + H]+.

Step 4: hydrochloric acid/ethyl acetate (4 M, 30 mL) was added to a dichloromethane (10 mL) solution containing Compound 1-4 (3.5 g, 8.2 mmol), and stirred at 25 ° C for 0.5 hours. Solid was precipitated, filtered, and dried to obtain Compound 1-5 (3.3 g of hydrochloride, crude product), which was directly used for the next reaction without purification.

LCMS (ESI) m/z: 324.1 [M+H]+.

Step 5: Pd/C (1 g, 10%) was added to a methanol (100 mL) solution containing Compound 1-5 (3.0 g, 8.34 mmol, hydrochloride) in a nitrogen atmosphere. The suspension was replaced three times with hydrogen, and then stirred at 30 °C in hydrogen atmosphere (30 psi) for 12 hours. The reaction solution was filtered and concentrated to obtain Compound 1-6 (3 g of hydrochloride, crude product), which was directly used for the next reaction without purification.

LCMS (ESI) m/z: 326.2 [M+H]+.

Step 6: Compound 1-6 (0.87 g, 2.40 mmol, hydrochloride) was dissolved in N, N-dimethylformamide (10 mL), added with HOBt (487 mg, 3.6 mmol,) and EDCI (691 mg, 3.6 mmol), then added with (1S) -2,2-difluorocyclopropyl formic acid (323 mg, 2.6 mmol) and diisopropylethylamine (621 mg, 4.8 mmol), and reacted at 15 °C for 12 hours. The reaction solution was concentrated under reduced pressure, and the residue was obtained by preparative HPLC (neutral system) to obtain the compound of formula (II).

1H NMR (400MHz, CD3OD) δ 7.32-7.73 (m, 2H), 6.95 (br s, 1H), 3.62-4.22 (m, 4H), 3.45 (br s, 1H), 3.18-3.37 (m, 1H), 2.61 (br s, 1H), 1.45-2.27 (m, 10H), 0.78-1.17 (m, 4H)_{∘} LCMS (ESI) m/z: 430.0[M + H]+

### Example 21 Preparation methods for individual crystal forms

50 mg Compound of formula (II) was weighed, added into a 2.0 mL glass vial, and added with 0.4 mL solvent mixture of methanol and water (volume ratio 1:1) to obtain a suspension, which was added with a magneton, and stirred on a heated magnetic stirrer (40 °C. After stirring for 100 hours, the suspended sample was centrifuged and placed overnight in a 35 °C vacuum drying oven. After drying, the sample was tested for XRPD (as shown in FIG. 6), which was determined to be the crystal form A of compound (II), while DSC (as shown in FIG. 7) was further tested.

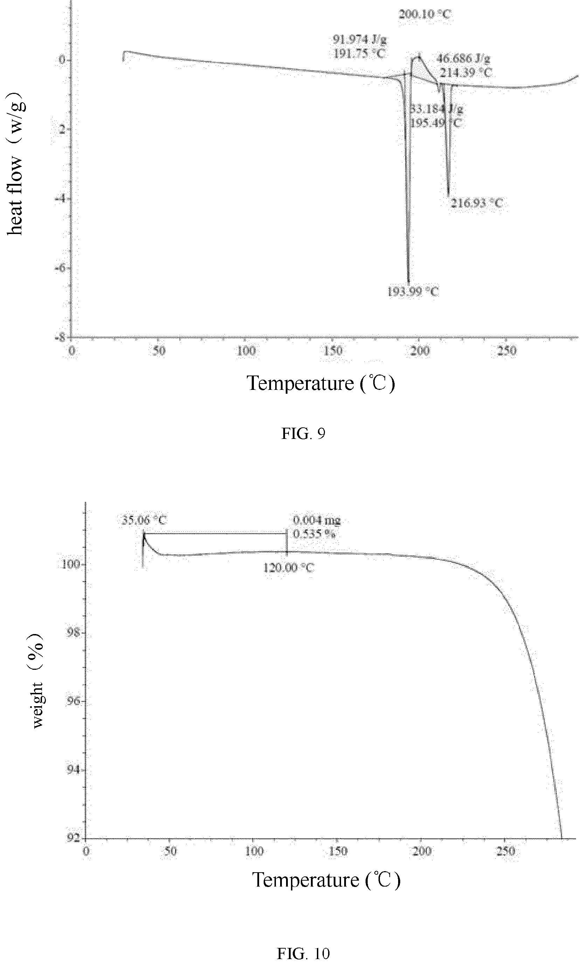

Approximately 50 mg of compound of formula (II) was weighed and added to a 2.0 mL glass vial, and added with 0.4 mL of ethyl acetate. After adding a magneton, the above sample was stirred in a heated magnetic stirrer (40 °C). After stirring for 100 hours, the suspended sample was centrifuged and placed overnight in a 35 °C vacuum drying oven. After drying, the sample was tested for XRPD (as shown in FIG. 8), which was determined to be the crystal form B of compound (II), while DSC (as shown in FIG. 9) and TGA (as shown in FIG. 10) were further tested.

The crystal form A of the compound of formula (II) was heated to 170 °C. It was determined via XRPD detection (as shown in FIG. 11) that the crystal form was changed. The new crystal form obtained was crystal form C of the compound of formula (II), while DSC was further detected (as shown in FIG. 12).

About 50 mg compound of formula (II) was weighed and added into a 2.0 mL glass vial, and added with 0.4 mL solvent mixture of ethanol and water (volume ratio 1:1) to obtain a suspension. After adding a magneton, the above sample was stirred on a heated magnetic stirrer (40 °C). After stirring for 100 hours, the suspended sample was centrifuged and placed overnight in a 35 °C vacuum drying oven. After drying, the sample was tested for XRPD (as shown in FIG. 13) and determined to be the crystal form D of the compound of formula (II).

### Example 22 Study on solid stability of crystal form B of compound of formula (II)

About 5mg of crystal form B was accurately weighed and placed in a dry and clean glass bottle, and spread into a thin layer to be used as a normal test sample. It was subjected to the influence factor test conditions (60 °C, 92.5% RH) and accelerated conditions (40 °C/75% RH and 60 °C/75% RH), and the sample was completely exposed. It was covered with aluminum foil and tie small holes. Sampling was taken for analysis at 5th day and 10th day. The sample placed under light conditions (visible light 1200000Lux, UV 200W) was fully exposed at room temperature.

The experimental results showed that the crystal form did not change under the influencing factors (high temperature -60 °C, high humidity -92.5% RH, light) and acceleration conditions (40 °C/75% RH and 60 °C/75% RH).

### Example 23 Study on the Biomedia Solubility of crystal form B of Compound of Formula (II)

### 1. Biomedia solubility experiment of crystal form B

Approximately 2 mg of sample crystal form B was weighed and placed into the sample bottle, and then added with 1.0 mL of different solvents [pure water, SGF (simulating gastric juice), FaSSIF (simulating intestinal fluid in fasting state), FeSSIF (simulating intestinal fluid in eating state)] in each bottle, and shaken well. It was placed on a constant temperature oscillator and shaken at 37 °C. After shaking for 24 hours, centrifuging was performed to obtain supernatant, which was separated and tested in terms of solubility. The supernatant (diluent ACN/H2O (1/1)) was diluted by a certain times (the solubility of the compound was low, and the supernatant was diluted twice except for SGF, and SGF was diluted 10 times), and the concentration was determined by using HPLC.

### 2. Preparation of diluent and mobile phase

Diluent: acetonitrile: water of 1:1. Mobile phase A: 0.1% TFA aqueous solution, for example: 1 mL of TFA was transferred into 1 L of pure water, mixed well, and degased by ultrasound. Mobile phase B: 100% acetonitrile.

### 3. Preparation of reference and sample solutions

Preparation of STD solution: crystal form B was used as the reference substance. Approximately 5 mg of the reference substance was weighed and added into a glass bottle, dissolved with 10 mL of diluent, sonicated for about 10 minutes to fully dissolve the sample, cooled to room temperature, and shaken well. Two copies were prepared in parallel and labeled as corresponding STD1 and STD2. The corresponding STD1 was diluted by diluent 10, 100, 1000, and 2000 times, and a standard curve was obtained for testing.

Preparation of sample solution: the supernatant (diluent ACN/H2O (1/1)) was diluted by a certain time (the solubility of the compound is small, and the supernatant is diluted twice except for SGF, and SGF was diluted by 10 times), shaken well, and placed it in a 1.5 mL liquid phase vial for testing. The concentration was determined by using HPLC.

### 4. Biomedia solubility results (as shown in Table 13)

**Table 13 Biomedia Solubility Results of crystal form B**

| Buffer | H2O | SGF | FeSSIF | FaSSIF |
|---|---|---|---|---|
| pH (24h) | 7.02 | 1.91 | 5.09 | 6.52 |
| Solubility (mg/ml)_24hr | 0.211 | 0.727 | 0.166 | 0.214 |
| LOQ=0.0001267mg/ml; Y=7778034.55X-1509.46 | | | | |

**Experimental conclusion:** The solubility of crystal form B in simulated biological media is good, which is conducive to obtaining good in vivo bioavailability.

## Claims

1. An oral preparations comprising a JAK inhibitor, **characterized in that**, the oral preparations comprises the JAK inhibitor and a pharmaceutical excipient, the JAK inhibitor comprises a compound of formula (I), an isomer thereof, pharmaceutically acceptable salts thereof, or crystal form A thereof: 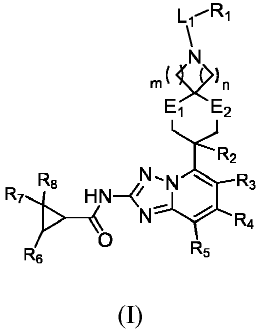 wherein,
E₁ and E₂ are independently selected from single bond, -CH₂- or -(CH₂)₂-, respectively;
L₁ is selected from single bond, - (CH₂)_{g}-, -C(=O)- or -C(=O)-(CH₂)ₕ-;
m is 1 or 2;
n is 1 or 2;
g is 1, 2 or 3;
h is 1, 2 or 3;
R₁ is selected from H, CN, C₁₋₆ alkyl or 3-6-membered cycloalkyl, wherein the C₁₋₆ alkyl and 3-6-membered cycloalkyl are optionally substituted by 1, 2 or 3 Rₐ;
R₂ is selected from H, F, Cl, Br, I or C₁₋₃ alkyl, wherein the C₁₋₃ alkyl is optionally substituted by 1, 2 or 3 R_{b};
R₃, R₄ and R₅ are independently selected from H, F, Cl, Br, I or C₁₋₃ alkyl, respectively, wherein the C₁₋₃ alkyl is optionally substituted by 1, 2 or 3 R_{c};
R₆, R₇ and R₈ are independently selected from H, F, Cl, Br, I or C₁₋₃ alkyl, respectively, wherein the C₁₋₃ alkyl is optionally substituted by 1, 2 or 3 R_{d};
Each Rₐ is independently selected from H, F, Cl, Br, I, CN or C₁₋₃ alkyl, respectively, wherein the C₁₋₃ alkyl is optionally substituted by 1, 2 or 3 R;
Each R_{b} is independently selected F, Cl, Br or I;
Each R_{c} is independently selected F, Cl, Br or I;
Each R_{d} is independently selected F, Cl, Br or I; and
Each R is independently selected F, Cl, Br or I,
wherein the pharmaceutical excipients comprise a filler, a disintegrant, an adhesive, a lubricant or a surfactant, or a combination of two or more thereof.

2. The oral preparation according to claim 1, **characterized in that**, the compound of formula (I), isomers thereof, or pharmaceutically acceptable salts thereof, wherein each Rₐ is independently selected from H, F, Cl, Br, I, or CN.

3. The oral preparation according to claim 1 or 2, **characterized in that**, the compound of formula (I), isomers thereof, or pharmaceutically acceptable salts thereof, wherein R₁ is selected from H, CN, C₁₋₃ alkyl or 3-5-membered cycloalkyl, wherein the C₁₋₃ alkyl and 3-5-membered cycloalkyl are optionally substituted by 1, 2, or 3 Ra.

4. The oral preparation according to claim 3, **characterized in that**, the compound of formula (I), isomers thereof, or pharmaceutically acceptable salts thereof, wherein R₁ is selected from H, CN, CH3, 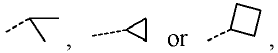 wherein CH₃, 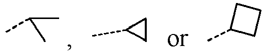 is optionally substituted by 1, 2, or 3 Ra.

5. The oral preparation according to claim 4, **characterized in that**, the compound of formula (I), isomers thereof, or pharmaceutically acceptable salts thereof, wherein R₁ is selected from H, CN, CF₃, CHF₂,

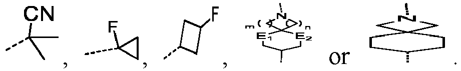

6. The oral preparation according to claim 1, **characterized in that**, the compound of formula (I), isomers thereof, or pharmaceutically acceptable salts thereof, wherein R2 is selected from H, F, Cl, Br, or I.

7. The oral preparation according to claim 1, **characterized in that**, the compound of formula (I), isomers thereof, or pharmaceutically acceptable salts thereof, wherein R₃, R₄, and R₅ are independently selected from H, F, Cl, Br, or I.

8. The oral preparation according to claim 1, **characterized in that**, the compound of formula (I), isomers thereof, or pharmaceutically acceptable salts thereof, wherein R₆, R₇, and R₈ are independently selected from H, F, Cl, Br, or I.

9. The oral preparation according to claim 1, **characterized in that**, the compound of formula (I), isomers thereof, or pharmaceutically acceptable salts thereof, wherein L₁ is selected from a single bond, -CH₂-, -(CH₂)₂-, -C(=O)- or -C(=O)-(CH₂)-.

10. The oral preparation according to claim 1, **characterized in that**, the compound of formula (I), isomers thereof, or pharmaceutically acceptable salts thereof, wherein the structural unit 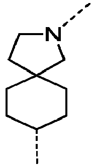 is selected from

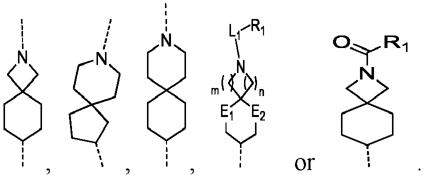

11. The oral preparation according to claim 1, **characterized in that**, the compound of formula (I), isomers thereof, or pharmaceutically acceptable salts thereof, wherein the structural unit 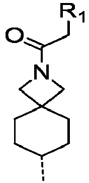 is selected from

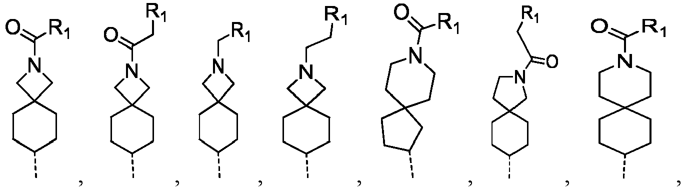

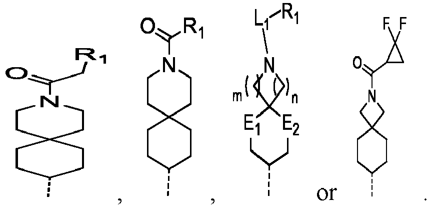

12. The oral preparation according to claim 1, **characterized in that**, the compound of formula (I), isomers thereof, or pharmaceutically acceptable salts thereof, wherein the structural unit 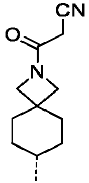 is selected from

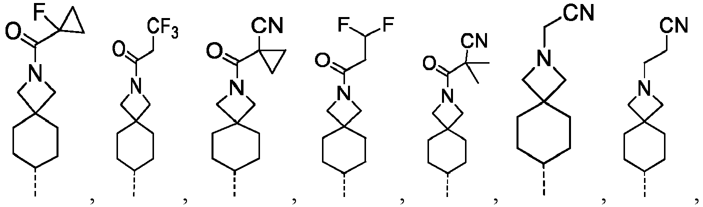

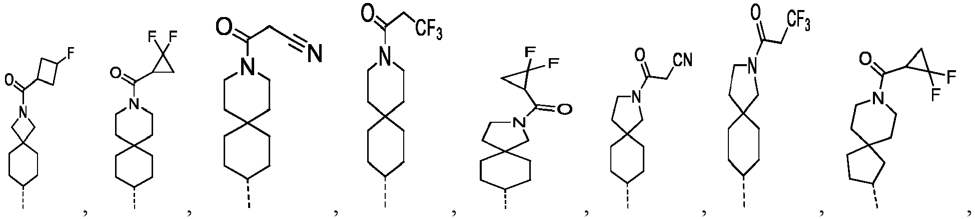

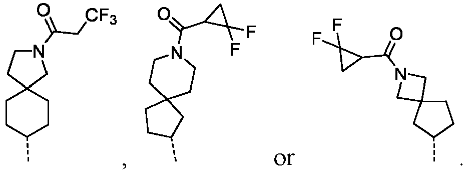

13. The oral preparation according to any one of claims 1-9, **characterized in that**, the compound of formula (I), isomers thereof, or pharmaceutically acceptable salts thereof, is selected from 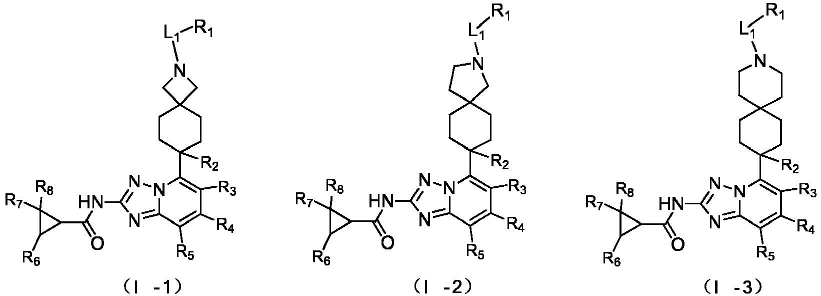 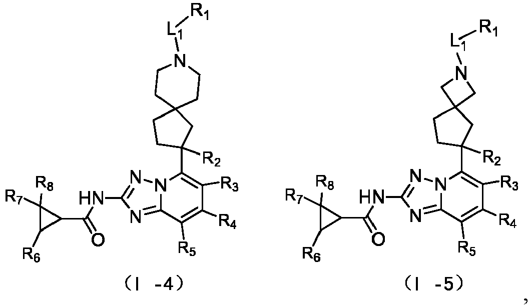 where
L₁ is defined in claim 1 or 9;
R₁ is defined in claims 1-5;
R₂ is defined in claim 1 or 6;
R₃, R₄, and R₅ is defined in claim 1 or 7; and
R₆, R₇, and R₈ is defined in claim 1 or 8.

14. The oral preparation according to claim 13, **characterized in that**, the compound of formula (I), isomers thereof, or pharmaceutically acceptable salts thereof, is selected from 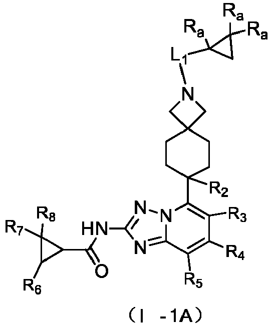 where,
L₁ is defined in claim 1 or 9;
R₁ is defined in claim 1 or 2;
R₂ is defined in claim 1 or 6;
R₃, R₄, and R₅ are defined in claim 1 or 7;
R₆, R₇, and R₈ are defined in claim 1 or 8.

15. An oral preparation comprising a JAK inhibitor, **characterized in that**, the JAK inhibitor comprises the following compounds, their isomers, or pharmaceutically acceptable salts thereof

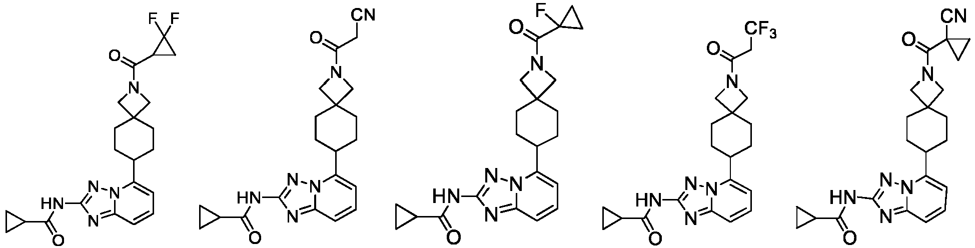

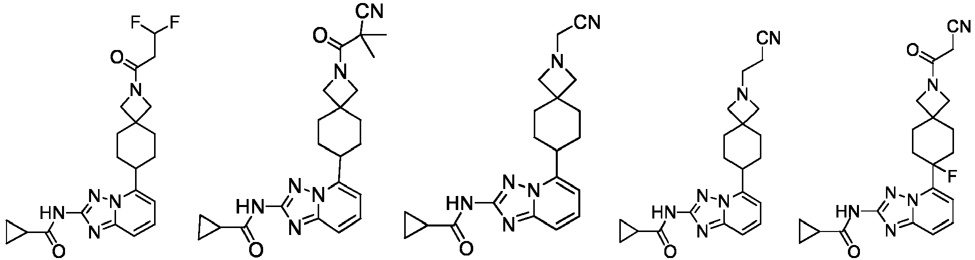

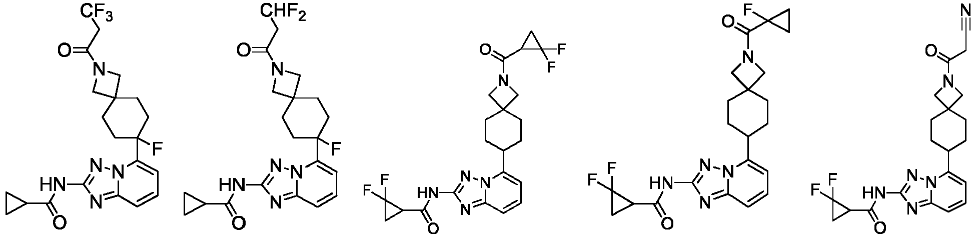

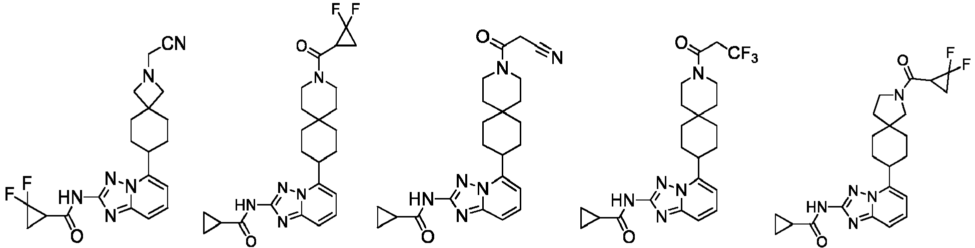

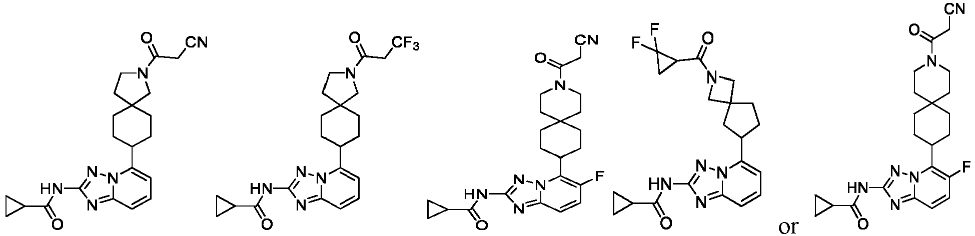

16. The oral preparation according to claim 15, **characterized in that**, the compound of formula (I), isomers thereof, or pharmaceutically acceptable salts thereof, is selected from 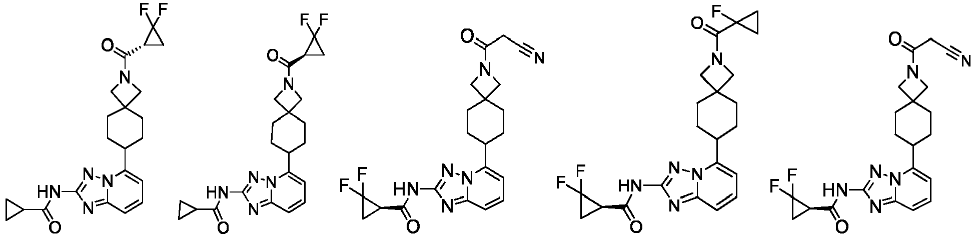 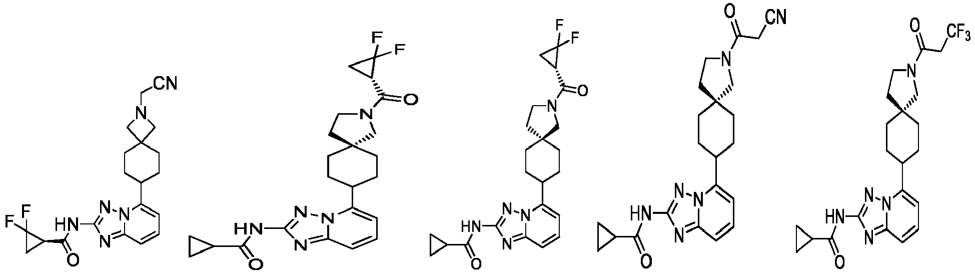 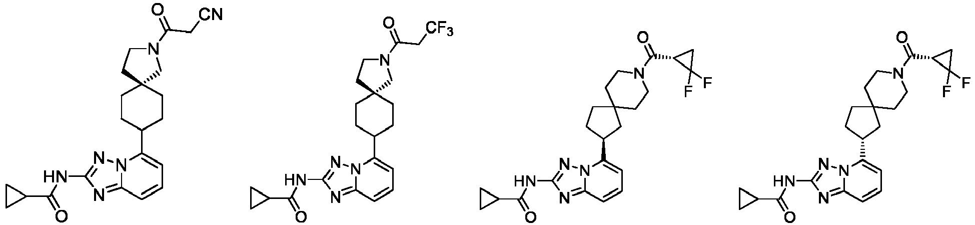 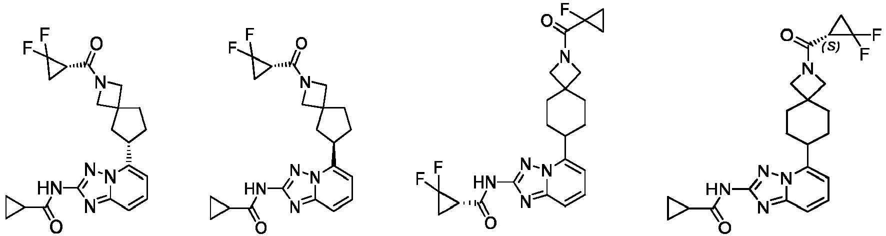 or

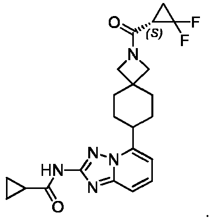

17. The oral preparation according to any one of claims 1-16, **characterized in that**, the oral preparation comprises one or more fillers.

18. The oral preparation according to any one of claims 1-16, **characterized in that**, the oral preparation comprises one or more disintegrants.

19. The oral preparation according to any one of claims 1-16, **characterized in that**, the oral preparation comprises one or more adhesives.

20. The oral preparation according to any one of claims 1-16, **characterized in that**, the oral preparation further comprises one or more lubricants.

21. The oral preparation according to any one of claims 1-16, **characterized in that**, the oral preparation further comprises one or more surfactants.

22. The oral preparation according to any one of claims 1-16, **characterized in that**, the oral preparation comprises one or more fillers and one or more disintegrants.

23. The oral preparation according to any one of claims 1-16, **characterized in that**, the oral preparation comprises one or more fillers, one or more disintegrants, and one or more adhesives.

24. The oral preparation according to any one of claims 1-16, **characterized in that**, the oral preparation comprises one or more fillers, one or more disintegrants, one or more adhesives, and one or more lubricants.

25. The oral preparation according to any one of claims 1-16, **characterized in that**, the oral preparation comprises one or more fillers, one or more disintegrants, one or more adhesives, one or more lubricants, and one or more surfactants.

26. The oral preparation according to any one of claims 17-25, **characterized in that**, the filler is selected from a group consisting of microcrystalline cellulose, lactose, pre-gelatinized starch or anhydrous dicalcium phosphate, or a combination of two or more of them.

27. The oral preparation according to any one of claims 18-26, **characterized in that**, the disintegrant is selected from a group consisting of cross-linked carboxymethyl cellulose sodium, carboxymethyl starch sodium, cross-linked polyvinylketone or dry starch, or a combination of two or more of them.

28. The oral preparation according to any one of claims 19-25, **characterized in that**, the adhesive is selected from a group consisting of carboxymethyl cellulose, hydroxypropyl cellulose, polyvinylketone, methyl cellulose, or ethyl cellulose, or a combination of two or more of them.

29. The oral preparation according to any one of claims 20-25, **characterized in that**, the lubricant is selected from a group consisting of magnesium stearate, micronized silica gel, talc powder, hydrogenated vegetable oil, polyethylene glycol or sodium dodecyl sulfate, or a combination of two or more of them.

30. The oral preparation according to any one of claims 20-25, **characterized in that**, the surfactant is sodium dodecyl sulfate.

31. An oral preparation according to any one of claims 1-30, **characterized in that**, the oral preparation comprises a JAK inhibitor, one or more fillers, one or more disintegrants, one or more adhesives, one or more lubricants, and one or more surfactants, the JAK inhibitor is a compound of structural formula (II), a pharmaceutically acceptable salt thereof, or a crystal form thereof,

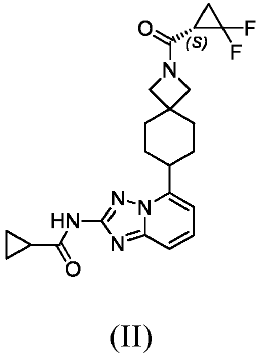

32. The oral preparation according to claim 31, **characterized in that**, the content of the JAK inhibitor is 1% to 50%, preferably 5% to 40%, further preferably 6% to 30%, more preferably 8% to 25%, most preferably 10%, 15%, and 20% of the total weight of the composition.

33. The oral preparation according to claim 31, **characterized in that**, the one or more fillers are selected from at least one of microcrystalline cellulose, lactose, pre-gelatinized starch, and anhydrous dicalcium phosphate.

34. The oral preparation according to claim 33, **characterized in that**, the content of microcrystalline cellulose in the filler is 0% to 78.5%, preferably 10% to 70%, further preferably 20% to 60%, more preferably 30% to 50%, most preferably 31.75%, 37.25%, 39.25%, 41.75%, 42.25%, and 43.75% of the total weight of the composition.

35. The oral preparation according to claim 33, **characterized in that**, the content of lactose in the filler is 0% to 78.5%, preferably 10% to 70%, further preferably 20% to 60%, more preferably 30% to 50%, most preferably 31.75%, 37.25%, 39.25%, 41.75%, 42.25%, and 43.75% of the total weight of the composition.

36. The oral preparation according to claim 33, **characterized in that** the content of pre-gelatinized starch in the filler is 0% to 78.5%, preferably 10% to 70%, further preferably 20% to 60%, more preferably 30% to 50%, and most preferably 39.25% of the total weight of the composition.

37. The oral preparation according to claim 33, **characterized in that**, the content of anhydrous calcium phosphate in the filler is 0% to 78.5%, preferably 10% to 70%, further preferably 20% to 60%, more preferably 30% to 50%, and most preferably 39.25% of the total weight of the composition.

38. The oral preparation according to claim 33, **characterized in that** the filling agent can be a mixture of lactose and microcrystalline cellulose, with a weight ratio of 0:1, 5:3, 3:1, 1:1, 1:0 for lactose and microcrystalline cellulose; preferably 1: 1.

39. The oral preparation according to claim 32, which is **characterized in that** the one or more disintegrants are selected from at least one of cross-linked carboxymethyl cellulose sodium, carboxymethyl starch sodium, cross-linked povidone and dry starch.

40. The oral preparation according to claim 39, which is **characterized in that**, the content of cross-linked carboxymethyl cellulose sodium of the disintegrant is 2%~16%, preferably 4%, 8%, and 12% of the total weight of the composition.

41. The oral preparation according to claim 39, **characterized in that**, the content of the disintegrating agent sodium carboxymethyl starch is 2% to 16%, preferably 8% of the total weight of the composition.

42. The oral preparation according to claim 39, **characterized in that**, the content of the disintegrating agent cross-linked polyvinylketone is 2% to 16%, preferably 8% of the total weight of the composition.

43. The oral preparation according to claim 32, **characterized in that**, the one or more adhesives are selected from at least one of carboxymethyl cellulose, hydroxypropyl cellulose, povidone, methyl cellulose, and ethyl cellulose.

44. The oral preparation according to claim 43, which is **characterized in that**, the content of the adhesive Hypromellose is 0.1%~5%, preferably 1%~4%, more preferably 2%~3%, most preferably 1.5% of the total weight of the composition.

45. The oral preparation according to claim 43, **characterized in that**, the content of the adhesive hydroxypropyl cellulose is 0.1% to 5%, preferably 1% to 4%, more preferably 2% to 3%, and most preferably 1.5% of the total weight of the composition.

46. The oral preparation according to claim 43, **characterized in that**, the content of the adhesive povidone is 0.1% to 5%, preferably 1% to 4%, more preferably 2% to 3%, and most preferably 1.5% of the total weight of the composition.

47. The oral preparation according to claim 32, **characterized in that**, the said one or more lubricants are selected from magnesium stearate, micropowder silica gel, talcum powder, hydrogenated vegetable oil, polyethylene glycol and sodium dodecyl sulfate.

48. The oral preparation according to claim 47, **characterized in that** the content of the lubricant is 0.1% to 5%, preferably 0.5% to 3%, more preferably 1% of the total weight of the composition.

49. The oral preparation according to claim 32, **characterized in that**, the surfactant is selected from sodium dodecyl sulfate.

50. The oral preparation according to claim 49, **characterized in that**, the content of the surfactant is 0.1% to 5%; preferably 1% to 3%; most preferably 2% of the total weight.

51. The oral preparation according to claims 1-50, **characterized in that**, the crystal form of the compound or pharmaceutically acceptable salt of (II) is crystal form A, B, C, or D,

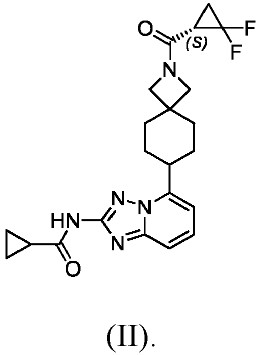

52. The oral preparation according to claim 51, **characterized in that**, the XRPD characteristic peaks of the crystal form A of the compound of formula (II) comprises:
| **No.** | **2θ angle (°)** | **Relative strength (%)** | **No.** | **2θ angle (°)** | **Relative strength (%)** |
|---|---|---|---|---|---|
| 1 | 6.91 | 100.0 | 18 | 25.88 | 8.3 |
| 2 | 10.34 | 13.7 | 19 | 26.18 | 4.9 |
| 3 | 12.21 | 64.7 | 20 | 26.93 | 2.8 |
| 4 | 13.69 | 23.1 | 21 | 27.52 | 18.9 |
| 5 | 14.44 | 9.8 | 22 | 28.73 | 2.6 |
| 6 | 16.11 | 3.1 | 23 | 29.12 | 8.2 |
| 7 | 17.44 | 12.2 | 24 | 29.92 | 6.8 |
| 8 | 18.11 | 18.7 | 25 | 31.56 | 6.9 |
| 9 | 18.76 | 11.7 | 26 | 31.86 | 9.6 |
| 10 | 19.06 | 76.7 | 27 | 32.43 | 6.0 |
| 11 | 19.86 | 32.6 | 28 | 32.64 | 7.2 |
| 12 | 20.59 | 28.8 | 29 | 33.04 | 2.5 |
| 13 | 22.06 | 24.8 | 30 | 33.30 | 2.3 |
| 14 | 22.63 | 6.8 | 31 | 34.52 | 10.0 |
| 15 | 23.49 | 2.4 | 32 | 35.21 | 3.9 |
| 16 | 24.46 | 12.0 | 33 | 35.56 | 2.8 |
| 17 | 25.27 | 4.6 | | | |

53. The oral preparation according to claim 51, **characterized in that**, the XRPD characteristic peaks of the crystal form B of the compound of formula (II) comprises:
| **No.** | **2θ angle (°)** | **Relative strength (%)** | **No.** | **2θ angle (°)** | **Relative strength (%)** |
|---|---|---|---|---|---|
| 1 | 5.13 | 65.7 | 20 | 26.06 | 9.0 |
| 2 | 7.34 | 43.4 | 21 | 26.61 | 4.1 |
| 3 | 10.14 | 27.3 | 22 | 27.03 | 7.2 |
| 4 | 10.56 | 23.7 | 23 | 27.69 | 2.0 |
| 5 | 11.46 | 13.7 | 24 | 28.45 | 2.5 |
| 6 | 11.72 | 42.7 | 25 | 28.97 | 5.5 |
| 7 | 14.64 | 2.1 | 26 | 29.49 | 8.8 |
| 8 | 16.67 | 27.0 | 27 | 29.81 | 2.8 |
| 9 | 16.86 | 13.6 | 28 | 30.41 | 6.7 |
| 10 | 18.84 | 22.5 | 29 | 30.72 | 3.4 |
| 11 | 19.14 | 55.0 | 30 | 31.15 | 3.2 |
| 12 | 20.31 | 3.7 | 31 | 32.00 | 3.1 |
| 13 | 21.18 | 100.0 | 32 | 32.35 | 3.0 |
| 14 | 21.78 | 20.5 | 33 | 33.34 | 3.6 |
| 15 | 22.03 | 9.3 | 34 | 33.65 | 2.3 |
| 16 | 22.54 | 14.2 | 35 | 37.00 | 2.5 |
| 17 | 22.96 | 13.2 | 36 | 38.60 | 2.8 |
| 18 | 24.52 | 4.1 | 37 | 39.66 | 2.1 |
| 19 | 25.27 | 11.5 | | | |

54. The oral preparation according to claim 51, **characterized in that**, the XRPD characteristic peaks of the crystal form C of the compound of formula (II) comprises:
| **No.** | **2θ angle (°)** | **Relative strength (%)** | **No.** | **2θ angle (°)** | **Relative strength (%)** |
|---|---|---|---|---|---|
| 1 | 5.76 | 19.3 | 8 | 18.66 | 29.0 |
| 2 | 8.92 | 31.2 | 9 | 19.17 | 22.1 |
| 3 | 11.50 | 13.9 | 10 | 20.26 | 100.0 |
| 4 | 14.28 | 11.4 | 11 | 22.98 | 6.2 |
| 5 | 16.35 | 23.6 | 12 | 24.79 | 24.0 |
| 6 | 17.54 | 11.4 | 13 | 29.77 | 5.1 |
| 7 | 17.99 | 7.5 | | | |

55. The oral preparation according to claim 51, **characterized in that**, the XRPD characteristic peaks of the crystal form D of the compound of formula (II) comprises:
| **No.** | **2θ angle (°)** | **Relative strength (%)** | **No.** | **2θ angle (°)** | **Relative strength (%)** |
|---|---|---|---|---|---|
| 1 | 7.12 | 100.0 | 14 | 21.42 | 44.1 |
| 2 | 10.28 | 12.3 | 15 | 21.84 | 7.9 |
| 3 | 10.68 | 2.7 | 16 | 23.95 | 3.0 |
| 4 | 12.45 | 36.0 | 17 | 24.50 | 5.1 |
| 5 | 13.04 | 7.2 | 18 | 25.74 | 4.3 |
| 6 | 14.28 | 26.7 | 19 | 26.69 | 4.9 |
| 7 | 14.64 | 30.4 | 20 | 27.24 | 2.5 |
| 8 | 16.06 | 3.1 | 21 | 28.72 | 17.0 |
| 9 | 17.30 | 7.2 | 22 | 29.49 | 5.0 |
| 10 | 17.50 | 10.8 | 23 | 31.80 | 4.3 |
| 11 | 18.31 | 16.1 | 24 | 34.05 | 7.8 |
| 12 | 20.54 | 77.4 | 25 | 36.04 | 6.1 |
| 13 | 20.93 | 20.5 | | | |

56. The oral preparation according to claim 52, **characterized in that**, the XRPD diagram of the crystal form A of the compound of formula (II) is shown in FIG. 6.

57. The oral preparation according to claim 53, **characterized in that**, the XRPD diagram of the crystal form B of the compound of formula (II) is shown in FIG. 8.

58. The oral preparation according to claim 54, **characterized in that**, the XRPD diagram of the crystal form C of the compound of formula (II) is shown in FIG. 11.

59. The oral preparation according to claim 52, **characterized in that**, the XRPD diagram of the crystal form D of the compound of formula (II) is shown in FIG. 13.

60. A preparation method for oral preparations according to any one of claims 1-59, wherein the preparation method is selected from one of wet granulation, dry granulation process, and powder direct pressing process, preferably the wet granulation.

61. The preparation method according to claim 60, wherein the filler, disintegrant, adhesive, lubricant, or surfactant is added internally or externally.

62. A use of the oral preparation according to any one of claims 1-59 in the preparation of drugs for treating autoimmune diseases, wherein the autoimmune diseases are preferably Rheumatoid arthritis, inflammatory bowel disease, and atopic dermatitis.
